# EUROPEAN PATENT SPECIFICATION

(11) **EP 3 700 590 B1**
(45) Date of publication and mention of the grant of the patent: **12.07.2023**
(21) Application number: 18789178.3
(22) Date of filing: 25.10.2018
(51) Int. Cl.: A61L 27/18

(54) **A COMPOSITION COMPRISING THIOL, ALKENE AND PHOSPHONIC ACID CONTAINING COMPOUNDS FOR USE AS A PRIMER FOR ADHESION IMPROVEMENT**
ZUSAMMENSETZUNG MIT THIOL, ALKEN UND PHOSPHONSÄURE MIT VERBINDUNGEN ZUR VERWENDUNG ALS PRIMER ZUR VERBESSERUNG DER HAFTUNG
COMPOSITION COMPRENANT DES COMPOSÉS CONTENANT DU THIOL, DE L'ALCÈNE ET DE L'ACIDE PHOSPHONIQUE DESTINÉE À ÊTRE UTILISÉE COMME APPRÊT POUR L'AMÉLIORATION DE L'ADHÉRENCE

(30) Priority: 25.10.2017 WO PCT/EP2017/077350
(43) Date of publication of application: 02.09.2020
(73) Proprietor: Biomedical Bonding AB, 114 28 Stockholm (SE)
(72) Inventor: MALKOCH, Mikael, 187 63 Täby (SE); GRANSKOG, Viktor, 182 32 Danderyd (SE); GARCIA GALLEGO, Sandra, 19005 Guadalajara (ES); ARSENEAULT, Mathieu, Québec Québec G1Y 3HC (CA)
(74) Representative: Brann AB
(86) International application number: PCT/EP2018/079289
(87) International publication number: WO 2019/081641

(56) References cited:
- WO-A1-2012/126695
- WO-A1-2018/077973
- WO-A2-2011/048077

## Description

### FIELD OF THE INVENTION

The present invention relates to a composition that may be used as a primer on bone or tooth. The primer composition provides good mechanical properties and may be applied also to wet bone. The invention further relates to an allyl-phosphonic acid containing compound. The present invention may be used in various applications and may be provided as a kit.

### BACKGROUND

With an increasing number of elderly people in the next decades it is estimated that the number of fractures all categories will increase substantially. Most fractures in the society are caused by accidents while a smaller amount is caused by diseases such as osteoporosis. Among the accidental factors including traffic, fall, leisure and violence, fractures due to fall is the most common causes. In Sweden statistics showed that fractures all categories was the most frequent cause to in-hospital treatment among people aged 65+. Within the next decades it can be foreseen that the pattern of fractures is similar all around the world as the demographic picture is the same in most countries. As a consequence, the health care costs will increase tremendously due to the need of in-hospital treatment. Thus, there is a need for the society to focus not only on primary prevention but also on new and effective fracture treatment aiming at reducing the number of fractures and the total in-hospital stay. The treatment of fractures all categories are conservative with no surgical procedures or with surgical stabilization with various tools. The most frequent surgical method is stabilizing the fractures by application of metals and screws made of titanium or stainless steel. Depending on the type and localization of the fractures, the surgical procedure is performed during either local or general anesthesiology. Although the accurate and good surgical results including fracture stabilization and healing, there is a demand for alternative material use.

For decades, researchers have focused on developing adhesive systems for bone fracture fixation and various approaches have been evaluated. However, the struggle with either poor adhesion or biocompatibility issues of the today available adhesive systems have delayed an expansion of the usage area further than the current use of bone cements. Recent research has come a long way in providing solutions for adhesive systems for wet environments with more biocompatible approaches and innovative methods. One promising fixation method is the Fiber Reinforced Adhesive Patch (FRAP), described in WO2011048077. The FRAP is applied over the fracture without entering the cross-section of the break, thus diminishing the interference with the healing of the fracture compared to other proposed adhesive fixation methods. The FRAP technology can enable a quick and mild fixation of various fractures that today are considered difficult due to sensitive locations, near joints or when drilling is not a possible option.

To tackle the difficulty in adhering to wet surfaces and then stay stable in the wet environment, the adhesive system may contain an adhesion-enhancing primer solution and a more hydrophobic adhesive matrix of the triazine-trione components tris[2-(3-mercaptopropionyloxy)ethyl] isocyanurate (TEMPIC) and 1,3,5-Triallyl-1,3,5-triazine-2,4,6(1H,3H,5H)-trione (TATATO). According to earlier studies, the adhesion-enhancing primer is very important for the final adhesive strength towards wet bone. The primer should both adhere to the wet bone surface and create a compatible surface and preferably have the capacity to covalently bond to the adhesive matrix. This approach has been used in dentistry for many years, where the use of acrylate monomers with acidic adhesion-enhancing groups of phosphoric, phosphonic or carboxylic acid have enabled self-etch adhesive systems. The acidic monomers dissolve hydroxyapatite and create ionic bonds to calcium ions forming precipitates with very low solubility that integrate within the hydroxyapatite surface. However, the use of acrylate polymerization systems is connected to cytotoxic leakage of unreacted monomers. Still, the use of such adhesion-enhancing groups is of high interest for bone adhesives due to the large fraction of hydroxyapatite in bone.

WO2012126695 discloses a stable curable thiol-ene composition suitable for preparing three dimensional objects. WO2012126695 aims at solving the problem of poor shelf life of curable thiol-ene compositions and discloses a composition comprising an ethylenically or acetylenically unsaturated monomer, a thiol, optionally a photoinitiator, a phosphonic acid and a component of benzene or naphthalene containing compound.

Adhesive systems for fixation of bone fractures are much needed as an alternative or complement to pins or screws for bone fractures. However, the wet environment in the body is challenging and many approaches struggle with either poor adhesion, biocompatibility issues or an inadequate fixation methodology. This has delayed an expansion of the clinical applications of adhesives for bone fractures.

### SUMMARY OF THE INVENTION

The object of the present invention is to overcome the drawbacks of the prior art. The present primers enable a high strength adhesive fixation method functioning in the wet environment present such as in bone repair applications. For dental restoratives, our primer could be vital for being able to implement use of thiol-ene resins which could reduce problems of leakage of monomers and curing shrinkage stress of today's acrylate resins. Thiol-ene resins would also distance dental restorations from use of bisphenol A derivatives which are known to be potentially harmful (as is monomer leakage e.g. from acrylates).

The present invention uses thiol-ene coupling polymerization and enables the use of thiol-ene resins in both bone and dental restorations. Thiol-ene resins have the benefits of being prepared through a benign and efficient reaction, insensitive to oxygen inhibition. Thiol-ene resins gelate at high conversions due to a stepwise network formation of flexible thioether linkages. A high gelation point reduces the curing induced shrinkage and increases the final conversion of the crosslinked network, which could lead to both better stability and minimal release of monomers from the restorations, compared to acrylate systems that today are used both as dental restoratives and bone cements.

### Aspects

In a first aspect the present invention relates to composition according to claim 1.

Further described, but not claimed is a composition for use as a primer for hard tissue such as bone or tooth, or metal or synthetic material.

Further described, but not claimed is a composition for use as a coating or a filler for hard tissue, or metal or synthetic material.

In a second aspect the present invention relates to a patch comprising
a first layer, also called primer, comprising the composition according to any one of claims 1 to 12;
a second layer of adhesive comprising photoinitiator, a Component A comprising at least two thiol groups, a Component B comprising vinyl reactive groups chosen from vinyl, acrylate, methacrylate, allyl and unsaturated cyclic vinyls, or alkynes and optionally fillers; and
Optionally a third layer comprising Component A comprising at least two thiol groups, a Component B comprising vinyl reactive groups chosen from vinyl, acrylate, methacrylate, allyl and unsaturated cyclic vinyls, or alkynes and fillers.

Described herein, but not claimed is a filler comprising the composition according to the present invention wherein the filler further comprises particles of a ceramic filler, such as a calcium phosphate, e.g. hydroxyapatite.

In a third aspect the present invention relates to a ceramic material, such as a calcium phosphate material, having a coating comprising the composition according to the present invention.

In a fourth aspect the present invention relates to a kit comprising at least two containers wherein any one container in the kit can contain any of
water;
a first compound having at least two thiol groups;
a second compound having the general structure of

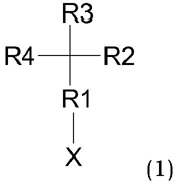

wherein R1 is an alkyl, a dialkyl ether, dialkyl ester, dialkyl urethane or a dialkyl amide group, R2 is an at least one allyl or alkyne containing group, R3 is hydrogen, an alkyl group or an at least one allyl containing group, or an alkyne group or an at least one alkyne containing group, and R4 is hydrogen, an alkyl group or an at least one allyl containing group, or an alkyne group or an at least one alkyne containing group;
wherein the first compound or the second compound comprises at least one phosphonic acid containing group X with proviso that when the second compound comprises the at least one phosphonic acid containing group said group is bound to the R1 group;
and a photo initiator.

### Various embodiments of the invention

An aqueous composition comprising
a first compound having at least two thiol groups;
a second compound having the general structure of 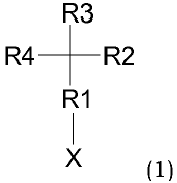
wherein R1 is an alkyl, a dialkyl ether, dialkyl ester, dialkyl urethane or a dialkyl amide group, R2 is an at least one allyl or alkyne containing group, R3 is hydrogen, an alkyl group or an at least one allyl containing group, or an alkyne group or an at least one alkyne containing group, and R4 is hydrogen, an alkyl group or an at least one allyl containing group, or an alkyne group or an at least one alkyne containing group;
wherein the first compound or the second compound comprises at least one phosphonic acid containing group X with the proviso that when the second compound comprises the at least one phosphonic acid containing group said group is bound to the R1 group; and
a third compound or additional compounds comprising at least one alkyne containing group;
a photo initiator.

The aqueous composition wherein the pH of the composition is in the range of 1.5 to 4.

The aqueous composition, wherein the molar ratio between the phosphonic acid groups and the allyles groups in the composition is 0.01 to 4 with alkynes as the third compound.

The aqueous composition wherein the molar ratio between the phosphonic acid groups and the allyl groups is 0.01-3 or 0.01-2 or 0.01-1 or 0.01 to 0.1 or 0.03 to 0.3 or 0.04 to 0.25 or 0.06 to 0.2 with alkynes as the third compound. The aqueous composition wherein the molar ratio between the phosphonic acid groups and the allyl groups is 0.01 to 0.1 with alkynes as the third compound.

The aqueous composition wherein the molar ratio between the phosphonic acid groups and the thiols groups is 0.01 to 0.5 with alkynes as the third compound.

The aqueous composition wherein the molar ratio between the phosphonic acid groups and the alkyne groups is 0.02 to 1 with alkynes as third compound or as second and third compounds.

The aqueous composition wherein the molar ratio between the alkene and/or allyl groups and alkyne groups is 0.02 to 2 with alkynes as third compound.

The aqueous composition wherein R1 is a C1-C10 alkyl group such as a C1-C6 alkyl group and wherein R2 is an alkyl allyl group or an alkoxy allyl group or alkyl alkyne group or an alkoxy alkyne group.

The aqueous composition wherein the second compound has the general structure (1) and wherein X is a phosphonic acid group.

The aqueous composition wherein each of the at least one allyl containing group is an alkyl allyl group containing two or more allyl groups, or each of the at least one alkyne containing group is an alkyl alkyne group containing one or more alkyne groups, or each of the at least one allyl containing group is a dialkyl ether allyl group containing two or more allyl groups or each of the at least one alkyne containing group is a dialkyl ether alkyne group containing one or more alkyne groups.

The aqueous composition wherein the second compound is a dendrimer having four or more allyl groups or two or more alkyne groups.

The aqueous composition wherein the third compound or additional compounds is a dendrimer having two or more alkyne groups.

The aqueous composition wherein the phosphonic acid containing group of the second compound has the structure of: 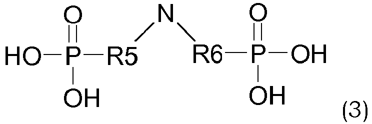 wherein R5 and R6 are individually an alkyl group such as a C1-C10 alkyl groups such as a methylene or an ethylene group.

The aqueous composition wherein the second compound comprises at least one phosphonic acid containing group and wherein the composition comprises a third compound having the general structure of 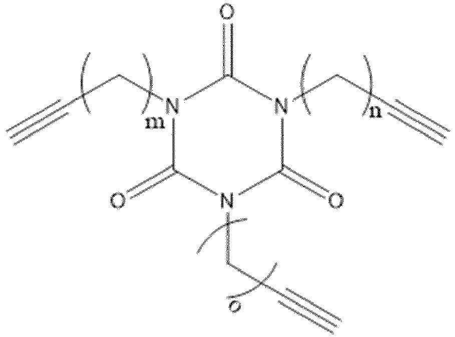 wherein m, n and o is 1 or higher.

The aqueous composition wherein the photo initiator is selected from lithium phenyl(2,4,6-trimethylbenzoyl)phosphinate (LAP), Irgacure 819, (2,4,6-trimethylbenzoyl)-phosphine oxide (BAPO), diphenyl(2,4,6-trimethylbenzoyl)phosphine oxide (TPO), camphorquinone, carboxylated camphorquinone.

The aqueous composition wherein the first compound is selected from tris 2-(3-mercaptopropionyloxy)ethyl isocyanurate (TEMPIC), ethoxylated-trimethylolpropane tri-3-mercaptopropionate (ETTMP), trimethylolpropane tri-3-mercaptopropionate, thiolated poly(ethylene glycol), 1,3,5-tris(3-mercaptopropyl)-1,3,5-triazinane-2,4,6-trione (TTTSH) and ethoxylated-trimethylolpropane tri-thiol (ETTT) and thiolated multi armed poly(ethylene glycol).

The aqueous composition wherein the composition further comprises ethanol or acetone preferably in an amount of 10-90wt% such as 40-60wt% of the total weight of the composition.

The aqueous composition wherein the composition comprises a third compound selected from 1,3,5-tri(prop-2-yn-1-yl)-1,3,5-triazinane-2,4,6-trione (TPYTATO), 1,3,5-tri(hex-5-yn-1-yl)-1,3,5-triazinane-2,4,6-trione (THYTATO), Bis(propynyloxymethyl)propanoic acid (BPPA).

The aqueous composition wherein the second compound has the general structure of 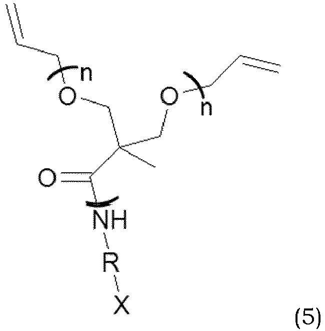 wherein n is 1 or higher, R is an alkyl group and X is a phosphonic acid containing group.

The aqueous composition wherein the total amount of the second compound and third compound is 1-10wt%, such as 2-6wt%, or 4-5wt%.

The aqueous composition further comprising a fourth compound or additional compounds comprising at least one allyl containing group or at least one thiol containing group or at least one alkyne.

The aqueous composition wherein the second compound comprises alkyl groups comprising alkene containing groups.

The composition for use as a primer for hard tissue such as bone or tooth, or for a metal or synthetic material.

The composition for use as a coating or a filler for hard tissue, or for a metal or synthetic material.

A patch comprising
a first layer (also called primer layer) comprising the composition according to the present invention;
a second layer of adhesive comprising photoinitiator, a Component A comprising at least two thiol groups, a Component B comprising vinyl reactive groups chosen from vinyl, acrylate, methacrylate, allyl and unsaturated cyclic vinyls, or alkynes; and
Optionally a third layer comprising Component A comprising at least two thiol groups, a Component B comprising vinyl reactive groups chosen from vinyl, acrylate, methacrylate, allyl and unsaturated cyclic vinyls, or alkynes.

The patch comprising a filler.

The patch wherein the filler is a ceramic filler.

The patch wherein the ceramic filler is in the third layer.

The patch according to claim 25 wherein the composition provided on the primer layer further comprises fibres.

A filler comprising the composition wherein the filler further comprises particles of a cermic filler material, such as calcium phosphate, e.g. hydroxyapatite.

A calcium phosphate material having a coating comprising the composition according to the present invention.

A kit comprising at least two containers wherein
any one container in the kit can contain any of
water;
a first compound having at least two thiol groups;
a second compound having the general structure of 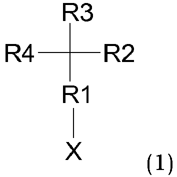
wherein R1 is an alkyl, a dialkyl ether, dialkyl ester, dialkyl urethane or a dialkyl amide group, R2 is an at least one allyl or alkyne containing group, R3 is hydrogen, an alkyl group or an at least one allyl containing group, or an alkyne group or an at least one alkyne containing group, and R4 is hydrogen, an alkyl group or an at least one allyl containing group, or an alkyne group or an at least one alkyne containing group;
wherein the first compound or the second compound comprises at least one phosphonic acid containing group X with proviso that when the second compound comprises the at least one phosphonic acid containing group said group is bound to the R1 group;
a third compound or additional compounds comprising at least one alkyne containing group;
and a photo initiator.

The kit wherein the kit is in the form of an at least two compartment syringe.

The kit wherein the kit comprises at least two additional containers wherein any one container in the kit can contain any of a photo initiator, a Component A comprising at least two thiol groups, a Component B comprising vinyl reactive groups chosen from vinyl, acrylate, methacrylate, allyl and unsaturated cyclic vinyls, or alkynes and optionally calcium phosphate particles or functionalized calcium phosphate particles;with proviso that the Compound A is not present in the same container as the Compound B.

The kit wherein the kit comprises calcium phosphate particles or functionalized calcium phosphate particles.

All the embodiments described herein relates to all the aspects unless otherwise stated.

### BRIEF DESCRIPTION OF THE FIGURES

Figure 1 shows the structure of molecules suitable as the first compound in the primer composition: a) Tris[2-(3-mercaptopropionyloxy)ethyl] isocyanurate, TEMPIC; b) Ethoxylated-Trimethylolpropan Tri(3-Mercaptopropionate), ETTMP; c) 1,3,5-tris(3-mercaptopropyl)-1,3,5-triazinane-2,4,6-trione, TTTSH; d) thiolated polyethylene glycol); e) Trimethylolpropane Tri(3-mercaptopropionate), TMPMP.

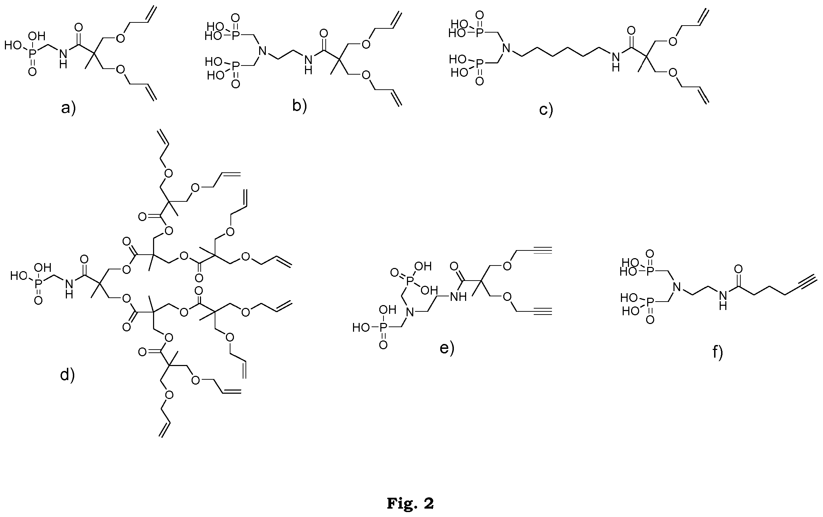

Figure 2 illustrates some examples of dendritic molecules suitable as the second compound in the primer composition: a) Bis(allyloxymethyl)methylpropanamido)methyl) phosphonic acid, Phn-G1-(ene)₂, b) Bis(allyloxymethyl)methylpropanamido)ethyl) azanediyl)bis(methylene))bis(phosphonic acid), (Phn)₂-EDA-G1-(ene)₂, c) (((6-bis(allyloxymethyl)methylpropanamido)hexyl) azanediyl)bis(methylene))bis(phosphonic acid), (Phn)₂-HDA-G1-(ene)₂, d) Phn-G3-(ene)s. e) (((2-(2-methyl-3-(prop-2-yn-1-yloxy)-2-((prop-2-yn-1-yloxy)methyl)propanamido)ethyl)azanediyl)bis(methylene))bis(phosphonic acid), (Phn)₂-EDA-G1-yne, f) (((2-(hex-5-ynamido)ethyl)azanediyl)bis(methylene))bis(phosphonic acid), (Phn)₂-EDA-G0-yne.
Figure 3 illustrates some examples of molecules suitable as the third compound or additional compounds in the primer composition: a) 1,3,5-tri(prop-2-yn-1-yl)-1,3,5-triazinane-2,4,6-trione (TPYTATO), b) 1,3,5-tri(hex-5-yn-1-yl)-1,3,5-triazinane-2,4,6-trione (THYTATO), c) Bis(propynyloxymethyl)propanoic acid (BPPA), d) 1,3,5-Triallyl-1,3,5-triazine-2,4,6(1H,3H,5H)-trione, TATATO; e) Trimethylolpropane diallyl ether (TMPDE); and f) Bis(allyloxymethyl)propanoic acid, BAPA.
Figure 4, shows the structure of suitable photoinitiators, Lithium phenyl(2,4,6-trimethylbenzoyl) phosphinate, LAP, b) Bis(mesitoyl)phenylphosphane oxide, Irgacure 819, c) Camphorquinone, CQ.

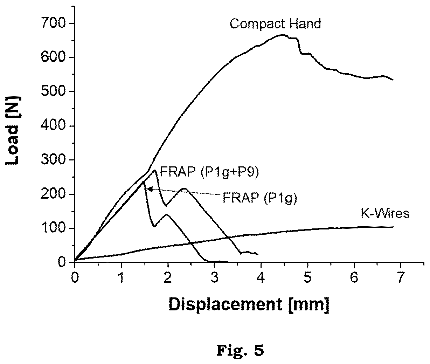

Figure 5, shows mechanical properties of the present invention.

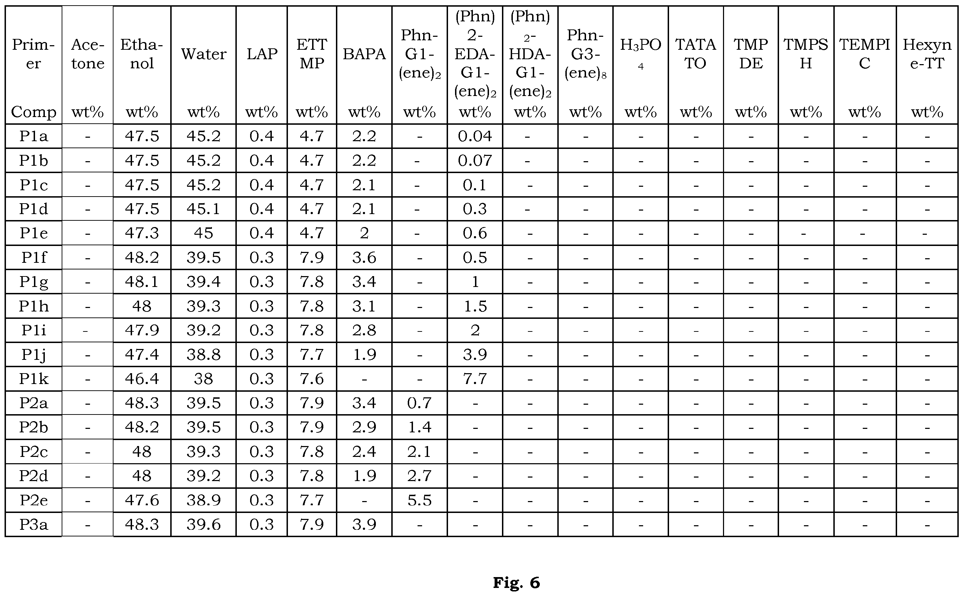

Figure 6 shows a table of primer compositions. Molecular structures are shown in figures 1-4.
Figure 7 shows a table of adhesive compositions.
Figure 8 shows a table of shear bond strength of primer and adhesive composite exhibiting different phosphonic acid/allyl ratios and pH. FRAP on bone is disclosed.
Figure 9 shows a table of shear bond strength of primer and adhesive composite. FRAP on bone is disclosed.
Figure 10 shows a table of shear bond strength of the present invention and pre-treatment. Adhesive on bone is disclosed.
Figure 11 shows mechanical properties of fixation methods. FRAP fixation of phalangeal fracture model is disclosed.
Figure 12 shows shear bond strength of pre-treatment and the primer and adhesive composite. Adhesive on dentin is disclosed.
Figure 13 shows a table of shear bond strength of primer and adhesive composite exhibiting different phosphonic acid/allyl ratios and pH. Adhesive on steel is disclosed.
Figure 14 shows a table of shear bond strength of primer and adhesive on porcine metacarpal comparing thiol-ene and thiol-yne compositions.
Figure 15 shows a table of shear bond strength of primer and adhesive on bone contaminated with dried blood serum.

### DETAILED DESCRIPTION OF THE INVENTION

The present inventors have shown that one of the most important components in fixation of tissue and material/patch adherence to tissue is a primer that increases the bond strength between the substrate and the tissue for example bone. The present invention is connected to the development of a Fiber Reinforced Adhesive Patch (FRAP) - a novel concept for adhesive fixation of bone fractures that could provide a new and innovative way of treating bone fractures and defects in a range of applications. The FRAP is further described in WO2011048077 and the present invention may be used in combination with the FRAP.

Until now the bond strength between a substrate and the tissue has not been sufficient for many applications such as bone restoration or fracture fixation. However the present inventors have developed new high performance primer components and primer compositions. These could solve the critical problem of getting high enough adhesion to tissue especially bone. Moreover, the primers also have additional applications (besides the FRAP concept) such as in other bone repair applications or void filling applications where adhesives need to be used and in dental applications where a major opportunity could exist in being able to improve the adhesion to tooth of dental repairs.

The concept is based on years' of research into the materials and their manufacturing. Mechanical shear tests and 3-point bending tests display that the primer can provide competing bond strength to today's dental adhesives towards both bone and tooth. The present primers enable superior phalangeal fracture fixations (compared to Kirschner wires) and can even compete with screw fixated metal plates. As an example we have benchmarked against the commercially available dental adhesive SE Bond which noted shear strength of 5.84 MPa on wet bone substrates.

Many commercially available dental primers or fixation compositions contains acrylates which from a toxicological point of view is not wanted. The present primers have shown unexpectedly high mechanical strength and the primers have shown high biocompatibility.

### The composition

The composition according to the present invention is an aqueous composition comprising a first compound having at least two thiol groups and a second compound having the general structure of 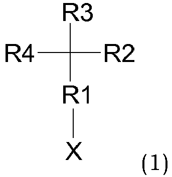 wherein R1 is an alkyl, a dialkyl ether, dialkyl ester, dialkyl urethane or a dialkyl amide group, R2 is an at least one allyl or alkyne containing group, R3 is hydrogen, an alkyl group or an at least one allyl containing group, or an alkyne group or an at least one alkyne containing group, and R4 is hydrogen, an alkyl group or an at least one allyl containing group, or an alkyne group or an at least one alkyne containing group. The alkyl groups of the second compound may comprise unsaturations in the form of an alkene, allyle and alkyne containing group, i.e. unsaturations inside (alkenes and alkynes) and at the end of the compounds (allyles and alkynes). In one embodiment, the compounds comprise unsaturations only at the end of the compounds. In another embodiment, the compounds comprise only unsaturations only inside the compounds. In a further embodiment, the compounds comprise a mix of unsaturations inside and at the end of the compounds. Furthermore the first compound or the second compound comprises at least one phosphonic acid containing group. This group when arranged on the second compound is bound to the R1 group of the general structure (1). The composition further comprises a photo initiator. The photo initiator may in an alternative embodiment be present in the adhesive composition. In one embodiment, the composition comprises a third compound or additional compounds comprising at least one allyl containing group or at least one thiol group or at least one alkyne containing group. In one embodiment, the third or additional compounds may be present in the primer composition. In another embodiment one or more of the third or additional compounds may be present in the adhesive composition.

The alkene groups used,
Each alkyne can react with two thiols and may thus increase the crosslinking density that leads to a stronger network compared to using allyl groups. The general ratio of phosphonic acid to allyl (alkene) is 0.01-4 with alkynes as the third compound. The following ratios are presently the preferred ones:
Phosphonic acid to allyl (alkene) of 0.01-1 with alkynes as the third compound;
Phosphonic acid to thiol of 0.01-0.5 with alkynes as the third compound;
Phosphonic acid to alkyne of 0.02-1 with alkynes as the third compound;
Phosphonic acid to alkyne of 0.02-1 with alkynes as the second and third compounds;
Alkene and/or allyl to alkyne 0.02-2 with alkynes as the third compound;
For metal substrates, molar ratios between phosphonic acid and allyls may also be 0.005 to 0.1 or 0.01 to 0.1.
For metal substrates, molar ratios between phosphonic acid and thiols may also be 0.005 to 0.1 or 0.01 to 0.1.
For metal substrates molar, ratios between phosphonic acid and alkynes may also be 0.01 to 0.1 or 0.01 to 0.1.

By varying the third compound, the number of allyl groups and alkyne groups may be in a molar ratio of 0.01 to 10 such as 0.02 to 5 or 0.02 to 2, such as 0.06 to 0.4 or 0.06 to 0.25. By using alkynes as the third compound the molar ratio of phosphonic acid groups and alkyne groups may be 0.01 to 1, such as 0.02 to 0.2. If alkynes are used as the second and third compound, the molar ratio between phosphonic acid groups and alkyne groups may be 0.01 to 1, such as 0.01 to 0.2 By having alkynes as the third and alternatively the second compound, the molar ratio between thiol groups and phosphonic acid groups may be 0.01 to 0.5, such as 0.01 to 0.3.

If alkynes are used as the second and the third compound, without allyl groups or other alkenes in the composition, the molar ratio between phosphonic acid groups and alkyne groups may be 0.01 to 1 such as 0.01 to 0.2 By having alkynes as the third, and alternatively the second compound, without allyls or other alkenes the molar ratio between thiol groups and phosphonic acid groups may be 0.01 to 0.5 such as 0.01 to 0.3.

Also the first, second and third or additional compounds are preferably at least partly soluble in water.

By varying the number of allyl groups and phosphonic acid groups on the first, second and the optional third or additional compounds the molar ratio between said functional groups may be varied and thereby the shear bond strength may be improved. The molar ratio may be varied by adding the different compounds in various amounts and/or by using compounds having multiple allyl groups or phosphonic acid groups. For example, by using a second or third compounds or additional compounds having a dendritic structure with alkyne containing groups. The molar ratio between the phosphonic acid groups and the allyl groups may be 0.01 to 4, e.g 0.01-3, 0.01-2, or 0.01-1, such as 0.01 to 0.1 or, 0.03 to 0.3 such as 0.04 to 0.25, or 0.06 to 0.20.

The ratio of the amount of phosphonic acid groups and the reactive groups for thiol-ene coupling was found to be a major factor in order to improve the bond strength to bone or metal. Phosphonic acid molecules are known to promote good bond strength to dentin and enamel when they are used in vinyl resins. However, phosphonic acid molecules are also well known radical scavengers, especially for thiyl (also named sulfenyl) radicals and are i.e. used as inhibitors in the thiol-ene resins disclosed in WO2012126695 and are supposed to work well even in amounts of 0.01wt%. In thiol-ene polymerization, it is important to yield high conversion to obtain a mechanically robust material and high bond strength and thus any radical scavenging effect is detrimental for achieving high bond strength with thiol-ene resins or thiol-yne resins. Allyls and alkenes reacts similarly to thiols with the main difference that an allyl only can react with one thiol and an alkyne can react with two thiols. Thus an unpredictably large increase in bond strength was obtained when certain allyls was replaced with alkynes in the primer as well, with an increased bond strength from 0.6 MPa for the P11 primer with only allyls as unsaturations to 5.6 MPa when the corresponding primer P10 with most allyl monomers replaced with an alkyne monomer was used on blood serum contaminated bone surface. An increase in bond strength from 9.1 MPa to 11 MPa was also seen when the alkyne containing primer P10 was used together with the thiol-yne adhesive composition A4 compared to using the P1f primer.

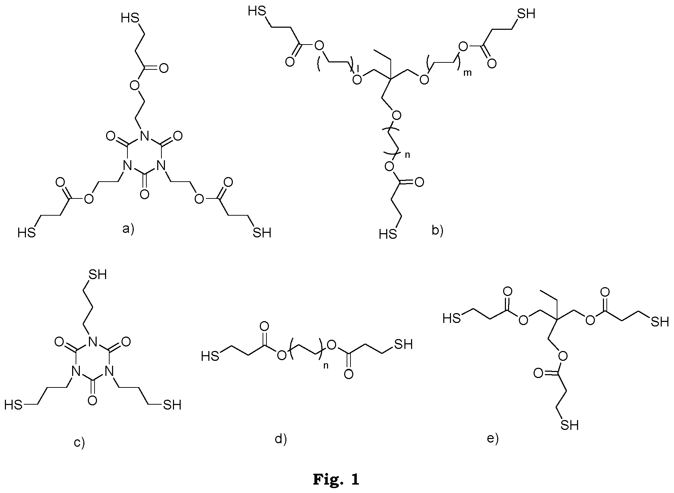

The first compound may be any suitable dithiol containing compound. The first compound may comprise at least three thiol groups, or at least four thiol groups. In figure 1 non-limiting examples of suitable first compounds are disclosed. In one embodiment the first compound is selected from tris 2-(3-mercaptopropionyloxy)ethyl isocyanurate (TEMPIC), ethoxylated-trimethylolpropane tri-3-mercaptopropionate (ETTMP), trimethylolpropane tri-3-mercaptopropionate (TMPMP), thiolated poly(ethylene glycol), 1,3,5-tris(3-mercaptopropyl)-1,3,5-triazinane-2,4,6-trione (TTTSH) and ethoxylated-trimethylolpropane tri-thiol (ETTT) and thiolated multi armed poly(ethylene glycol). In another embodiment the first compound is ETTMP. In one embodiment the amount of the first compound is 1 to 15wt% such as 3 to 12wt%, or 5 to 10wt% of the total composition.

The second compound may be any suitable allyl containing compound having the general structure (1). R1 may be a C1-C10 alkyl group such as a C1-C6 alkyl group. R2 may contain at least two allyl groups, or at least three, or at least 4 allyl groups or at least one alkyne groups, or at least two, or at least three, or at least 4 alkyne groups. In one embodiment R2 is an alkyl allyl group or an alkoxy allyl group, or an alkyl alkyne group or an alkoxy alkyne group. In another embodiment each of the at least one allyl containing group is an alkyl allyl group containing two or more allyl groups or each of the at least one alkyne containing group is an alkyl alkyne group containing one or more alkyne groups, or each of the at least one alkyne containing group is an alkyl alkyne group containing one or more alkyne groups, or each of the at least one allyl containing group is a dialkyl ether allyl group containing two or more allyl groups or each of the at least one alkyne containing group is a dialkyl ether alkyne group containing one or more alkyne groups. R3 may be a hydrogen, an alkyl group or an at least one allyl containing group or an alkyne group or an at least one alkyne containing group. In one embodiment R3 is an alkyl group such as a methyl group. In another embodiment R3 is an alkyl allyl group or an alkoxy allyl group or an alkyl alkyne group or an alkoxy alkyne group. In one embodiment R3 is an at least one allyl containing group containing two or more allyl groups or an at least one alkyne containing group containing one or more alkyne groups. R4 may be a hydrogen, an alkyl group or an at least one allyl containing group or an at least one allyl containing group. In one embodiment R4 is an alkyl group such as a methyl group. In another embodiment R4 is an alkyl allyl group or an alkoxy allyl group or an alkyl alkyne group or an alkoxy alkyne group. In one embodiment R4 is an at least one allyl containing group containing two or more allyl groups or an at least one alkyne containing group containing one or more alkyne groups. In one embodiment the second compound is a dendrimer having four or more allyl groups or two or more alkyne groups.

The second compound may comprise at least one phosphonic acid group and it is bound to the R1 group of the general structure (1). In one embodiment the second compound comprises two or more phosphonic acid groups. In one embodiment the phosphonic acid containing group of the second compound has the structure of: 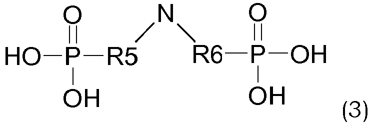 wherein R5 and R6 are individually an alkyl group such as a C1-C10 alkyl groups such as a methylene or an ethylene group.

In one embodiment the second compound is selected from the compounds disclosed in Figure 2, a) Bis(allyloxymethyl)methylpropanamido)methyl) phosphonic acid, Phn-G1-(ene)₂, b) Bis(allyloxymethyl)methylpropanamido)ethyl)azanediyl)bis(methylene))bis(phosphonic acid), (Phn)₂-EDA-G1-(ene)₂, c) (((6-bis(allyloxymethyl)methylpropanamido)hexyl) azanediyl)bis(methylene))bis(phosphonic acid), (Phn)₂-HDA-G1-(ene)₂, d) Phn-G3-(ene)₈. e) (((2-(2-methyl-3-(prop-2-yn-1-yloxy)-2-((prop-2-yn-1-yloxy)methyl)propanamido)ethyl)azanediyl)bis(methylene))bis(phosphonic acid), (Phn)₂-EDA-G1-yne, f) (((2-(hex-5-ynamido)ethyl)azanediyl)bis(methylene))bis(phosphonic acid), (Phn)₂-EDA-G0-yne.

In one embodiment the amount of the second compound is 0.1 to 15wt% such as 1 to 10wt%, or 2 to 8wt%, or 3 to 5wt% of the total composition. In one embodiment the amount of the second compound is 0.5 to 2.5wt%.

The third compound comprises at least one alkyne containing group. The third compound may have the general structure of 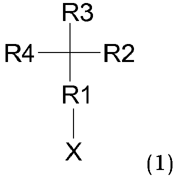 wherein R1 is an alkyl, a dialkyl ether, dialkyl ester, dialkyl urethane or a dialkyl amide group, R2 is an at least one allyl containing group or an at least one alkyne containing group, R3 is hydrogen, an alkyl group or an at least one allyl containing group or an at least one alkyne containing group and R4 is hydrogen, an alkyl group or an at least one allyl containing group or an at least one alkyne containing group.

R1 may be a C1-C10 alkyl group such as a C1-C6 alkyl group. R2 may contain at least two allyl groups, or at least three, or at least 4 allyl groups or at least two alkyne groups, or at least three, or at least 4 alkyne groups. In one embodiment R2 is an alkyl allyl group or an alkoxy allyl group or an alkyl alkyne group or an alkoxy alkyne group. In another embodiment each of the at least one allyl containing group is an alkyl allyl group containing two or more allyl groups or each of the at least one alkyne containing group is an alkyl alkyne group containing one or more alkyne groups, or wherein each of the at least one allyl containing group is a dialkyl ether allyl group containing two or more allyl groups or each of the at least one alkyne containing group is a dialkyl ether alkyne group containing one or more alkyne groups. R3 may be a hydrogen, an alkyl group or an at least one allyl containing group or an at least one alkyne containing group. In one embodiment R3 is an alkyl group such as a methyl group. In another embodiment R3 is an alkyl allyl group or an alkoxy allyl group or an alkyl alkyne group or an alkoxy alkyne group. In one embodiment R3 is an at least one allyl containing group containing two or more allyl groups or an at least one alkyne containing group containing one or more alkyne groups. R4 may be a hydrogen, an alkyl group or an at least one allyl containing group or an at least one alkyne containing group. In one embodiment R4 is an alkyl group such as a methyl group. In another embodiment R4 is an alkyl allyl group or an alkoxy allyl group or an alkyl alkyne group or an alkoxy alkyne group. In one embodiment R4 is an at least one allyl containing group containing two or more allyl groups or an at least one alkyne containing group containing one or more alkyne groups.

In one embodiment the third compound has the general structure of 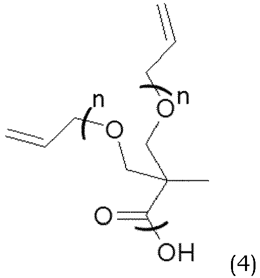 wherein n is 1 or higher. In one embodiment the third compound or additional compounds is! are a dendrimer having four or more allyl groups or two or more alkyne groups.

In one embodiment, the second compound comprises at least one phosphonic acid containing group and wherein the composition comprises a third compound having the general structure of 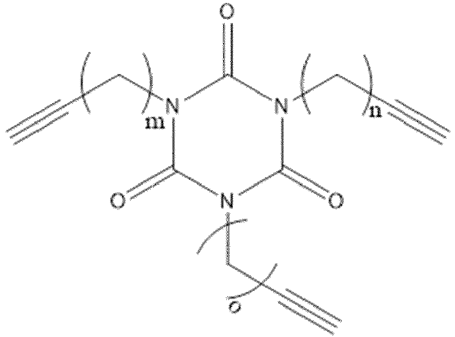 wherein m, n and o is 1 or higher.

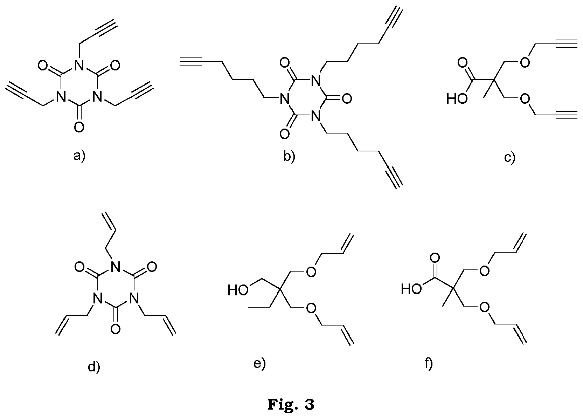

In one embodiment the third or additional compounds is the same compound as the second compound without the at least one phosphonic acid group. In one embodiment the third compound is selected from 1,3,5-tri(prop-2-yn-1-yl)-1,3,5-triazinane-2,4,6-trione (TPYTATO), 1,3,5-tri(hex-5-yn-1-yl)-1,3,5-triazinane-2,4,6-trione (THYTATO), Bis(propynyloxymethyl)propanoic acid (BPPA), 1,3,5-triallyl-1,3,5-triazine-2,4,6-trione (TATATO), trimethylolpropane diallyl ether (TMPDE) and bis(allyloxymethyl)propionic acid (BAPA), Figure 3. In one embodiment the amount of the third compound is 1 to 10wt% of the total composition, such as 2 to 7wt% or 3 to 5wt%. The amount is partly dependent on the wanted ratio between the phosphonic acid groups and allyl groups.

The total amount of allyl compounds in the composition is preferably 0.1 to 10wt%. The total amount of allyl compounds is the amount of the second compound. In exemplary embodiments, the total amount of allyl compounds is 0.1 wt% or more, or 1wt% or more, or 2wt% or more, or 3wt% or more, or 4wt% or more, but less than 10wt%, or 9wt% or less, or 8wt% or less, or 7wt% or less, or 6wt% or less, or 5wt% or less. In one embodiment the total amount of allyl compounds in the composition is around 4.5wt%.

The total amount of alkyne compounds in the composition is preferably 0.1 to 10wt%. The total amount of alkyne compounds is the amount of the second and third compounds. In exemplary embodiments, the total amount of alkyne compounds is 0.1 wt% or more, or 1wt% or more, or 2wt% or more, or 3wt% or more, or 4wt% or more, but less than 10wt%, or 9wt% or less, or 8wt% or less, or 7wt% or less, or 6wt% or less, or 5wt% or less. In one embodiment the total amount of alkyne compounds in the composition is around 4.5wt%.

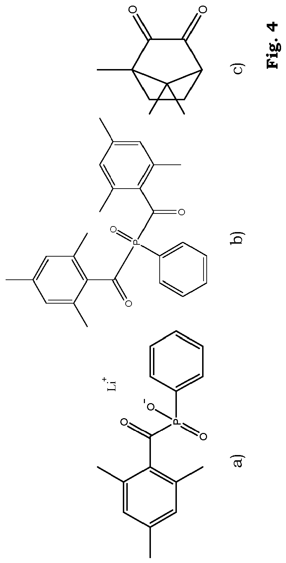

Any suitable photo initiator may be used. The initiator may be a peroxide, nitrile, phosphine oxide or quinone susceptible to radiation to create reactive species. In one embodiment the photo initiator is selected from lithium phenyl(2,4,6-trimethylbenzoyl)phosphinate(LAP), Irgacure 819, (2,4,6-trimethylbenzoyl)-phosphine oxide (BAPO), diphenyl(2,4,6-trimethylbenzoyl)phosphine oxide (TPO), camphorquinone, carboxylated camphorquinone. Figure 4 shows some photoinitiators. The amount of photo initiator may be 0.05 to 2wt% such as 0.1 to 1wt%.

The composition comprises water which is believed to activate the acid compounds by deprotonation. The water may be present in the form of a mixture of water and an alcohol such as methanol or ethanol, or as a mixture of water and acetone, or a mixture of water, an alcohol and acetone. The weight ratio between the water and the alcohol or acetone may be 1:1 to 1:100 (water: alcohol/acetone). The composition may comprise an alcohol or acetone in an amount of 10-90wt% such as 40-60wt% of the total weight of the composition. The amount of water in the composition may be 0.01 to 50wt% such as 0.5wt% or more, or 1 wt% or more, or 3wt% or more, or 5 wt% or more, or 8wt% or more, but 40wt% or less, or 30wt% or less, or 25wt% or less, or 20wt% or less, or 15wt% or less, or 10wt% or less. The composition is preferably essentially free from benzene or naphthalene compounds since they generally are toxic.

The aqueous composition according to the present invention may also comprise a fourth compound or further compounds comprising at least one allyl containing group or at least one thiol containing group or at least one alkyne.

### The allyl-phosphonic acid containing compound

The present application also relates to the use of a compound having the general structure of 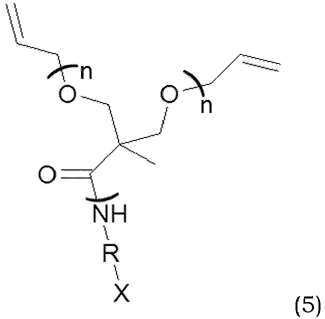 wherein n is 1 or higher, R is an alkyl group and X is a phosphonic acid containing group. R may be a C1-C10 alkyl group such as a C1-C6 alkyl group. When n is 2 or more the compound is a dendrimer. In one embodiment n is 2 or higher. In another embodiment n is 3 or higher, or 4 or higher. The phosphonic acid group X may be a phosphonic acid or a group having the general structure of 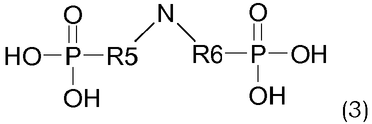 wherein R5 and R6 are individually an alkyl group such as a methylene or an ethylene group.

The compound may be used as a primer especially for hard tissue such as bone or tooth or metal or synthetic material. The compound may also be used as the second compound of the composition according to the present invention. It may also be used as a coating or a filler for a hard tissue or metal or synthetic material.

### Method of preparing

Here are some general non-limiting examples of how to prepare the present invention.

### Phn-G1-(ene)₂ (Figure 2a)

a. Activating BAPA (Figure 3c) in EtOAc by reacting with 1,1'-carbonyldiimidazole at 50°C for at least 30 minutes.
b. Adding Diethyl(aminomethyl)phosphonate and leave it to react at 50°C for 30 min.
c. Isolating the obtained product EtPhn-G1-(ene)₂.
d. Adding trimethylbromosilane in DCM until completion of reaction, evaporating and subsequently adding methanol.
e. Isolating the obtained product Phn-G1-(ene)₂ (Figure 2a).

### (Phn)₂-EDA-G1-(ene)₂ (Figure 2b)

a. Activating BAPA (Figure 3c) in EtOAc by reacting with 1,1'-carbonyldiimidazole at 50°C for at least 30 minutes.
b. Adding slowly ethylenediamine (EDA) and leave it to react at 50°C until reaction completion.
c. Isolating the obtained product EDA-G1-(ene)₂.
d. Adding p-formaldehyde in THF and reacting for at least 1 h at 70°C.
e. Adding dropwise dimethylphosphite and leave it to react at 70°C until reaction completion.
f. Isolating the obtained product MePhn)₂-EDA-G1-(ene)₂.
g. Adding trimethylbromosilane in DCM until completion of reaction, evaporating and subsequently adding methanol.
h. Isolating the obtained product (Phn)₂-EDA-Gl-(ene)₂ (Figure 2b).

### Phn-G3-(ene)₈ (Figure 2d)

a. Activating BAPA (Figure 3c) in EtOAc by reacting with 1,1'-carbonyldiimidazole at 50°C for at least 30 minutes.
b. Adding slowly bis-MPA dendron HOOC-G3-(OH)₄ and CsF and leave it to react at 50°C until reaction completion.
c. Isolating the obtained product HOOC-G3-(ene)s.
d. Activating compound HOOC-G3-(ene)₈ in EtOAc by reacting with 1,1'-carbonyldiimidazole at 50°C for at least 30 minutes.
e. Adding Diethyl(aminomethyl)phosphonate and leave it to react at 50°C for 30 min.
f. Isolating the obtained product EtPhn-G3-(ene)s.
g. Adding trimethylbromosilane in DCM until completion of reaction, evaporating and subsequently adding methanol.
h. Isolating the obtained product Phn-G3-(ene)₈ (Figure 2d).

### TPYTATO

a. Reacting cyanuric acid and propargyl bromide in DMF in the presence of K₂CO₃ for 16 h at 50°C.
b. Isolate the compound via extraction using DCM and water and finally recrystallization from a solution of 1: 1 mixture of methanol and chloroform

### THYTATO

a. Reacting cyanuric acid and 1-iodo-hexyne in DMSO in the presence of K₂CO₃ for 16 h at 23°C
b. Isolate the compound via extraction using ethyl acetate and water.

### Adhesive composite

In order to improve the mechanical properties of the primer, especially when used as a patch or a fixation material, an adhesive composite may be applied to the primer before any optional patch or fixation components are applied.

The adhesive composite comprises a photoinitiator, a Component A comprising at least two thiol groups, a Component B comprising vinyl reactive groups chosen from vinyl, acrylate, methacrylate, allyl, unsaturated cyclic vinyls, and alkynes, and optionally a filler, such as a ceramic filler, e.g. calcium phosphate particles or functionalized calcium phosphate particles. Preferably the Component B comprises vinyl reactive groups chosen from vinyl, allyl, unsaturated cyclic vinyls, and alkynes.

Compound A may be selected from the group consisting of pentaerythritol tetrakis(3-mercaptopropionate), trimethylolpropane tris(3-mercaptopropionate), , tris 2-(3-mercaptopropionyloxy)ethyl isocyanurate (TEMPIC) 1,3,5-tris(3-mercaptopropyl)-1,3,5-triazinane-2,4,6-trione, mercaptopropyl methylsiloxane-dimethylsiloxane copolymer,poly (mercaptopropyl) methylsiloxane, 2,2' (ethylenedioxy)diethanethiol, ditiotreitol, Tetraethyleneglycol-bis(3-mercaptopropionate), ethyleneglycol-bis(3-mercaptopropionate), trimethylolpropane diallylether, dipentaerytritolhexakis(3-merkaptopropionate), tetradecane-1,14-dithiol, ( +/-)-trans-1,2-bis (2-mercaptoacetamido) cyclohexane, (E)-S, S '-bis (lo-mercaptodecyl)-4,4'-(diazene-1,2-diyl) bis (4-cyanopentanethioate), bis(2-mercaptoethyl)sulfone, 2,5-dimercaptomethyl-1,4-dithiane, 1,4-butanediol-bis(3-mercaptopropionate), 1,16-hexadecanedithiol, undecane-1,11-dithiol, heptane-1,7-dithiol, 1,12-dimercaptododecane, octadecane-1,18-dithiol, (5-mercaptomethyl-2,4-dimethyl-phenyl)-methanethiol, (3-mercaptomethyl-5-methyl-phenyl)-methanetho, 1,2-benzenedimethanethiol, (4R, 5R)-4,5-bis (mercaptomethyl)-2,2-dimethyl-1,3-dioxolane, 3-bis (2-mercaptoethylthio)propane, ethanethiol, aceticacidmercapto-1,2,6-hexanetriyl ester, L-1,4-dithiothretol, glycerylthioglycolate, 3,6-dioxa-1,8-octanedithiol,trimethylolpropane-tris(mercaptoacetate) 2,3-butanediol-1,4-dimercapto-pentaerythritol-tetrakis(3-mercaptopropionate), ethanethiol-2,2',2"-nitrilotris, 2,2'-thiodiethanethiol, 1,9-nonanedithiol, 2,2'-oxydiethanethiol, and 10-decanedithiol.

Compound B may be selected from the group consisting of trimethylolpropane diallyl ether, 1,3,5-triallyl-1,3,5-triazine-2,4,6 (1H,3H,5H) -trione (TATATO), 1,3,5-tri(prop-2-yn-1-yl)-1,3,5-triazinane-2,4,6-trione (TPYTATO), b) 1,3,5-tri(hex-5-yn-1-yl)-1,3,5-triazinane-2,4,6-trione (THYTATO), trimethylolpropane diallyl ether, and poly(ethylene glycol) dimaleinimide.

The molar ratio between thiols and allyls of Compound A and Compound B respectively may be from 1:0.5 to 1:5 such as 1:1 to 1:3 or around 1:2. The molar ratio between thiols and alkynes of Compound A and B respectively may be 1:0.25 to 1:5 such as 1:0.3 to 1:3 or around 1:0.5.

Any suitable photo initiator may be used. The initiator in the adhesive composite may be the same as in the primer or it may be different and preferably it is at least partly soluble in the adhesive composite components. The initiator may be a peroxide, nitrile, phosphine oxide or quinone susceptible to radiation to create reactive species. In one embodiment the photo initiator is selected from lithium phenyl(2,4,6-trimethylbenzoyl)phosphinate (LAP), Irgacure 819, (2,4,6-trimethylbenzoyl)-phosphine oxide (BAPO), diphenyl(2,4,6-trimethylbenzoyl)phosphine oxide (TPO), camphorquinone, carboxylated camphorquinone.

The adhesive composite may comprise a filler, such as a ceramic filler, e.g. calcium phosphate particles such as hydroxyapatite. The calcium phosphate particles may be functionalized for example with a second compound molecule. The amount of calcium phosphate particles in the composite may be up to 99wt% such as 90wt% or less, or 80wt% or less, or 70wt% or less, or 60wt% or less, or 50wt% or less, or 1wt% or more, or 10wt% or more, or 20wt% or more, or 30wt% or more, or 40wt% or more. In one embodiment the amount of calcium phosphate particles is 35-65wt% or 50-60wt%.

In one embodiment, none of the second and third layers (i.e. the patch) comprise a filler. In another embodiment, the second and third layers of the adhesive comprise a filler, preferably a ceramic filler, e.g. calcium phosphate particles such as hydroxyapatite. In another embodiment, only the third layer of the adhesive comprises a filler, such as a ceramic filler.

The functionalized calcium phosphate particles may be prepared by mixing the functionalization agent with the particles in a solution. The functionalization agent may be a Second compound molecule and the solution may be an aqueous solution such as a mixture of an alcohol and water. A non-limiting example is disclosed in Example 2.

In one embodiment the amount of Compound A is 10-20wt%, the amount of Compound B is 20-35wt% and the amount of calcium phosphate particles or functionalized calcium phosphate particles is 40-70wt% with proviso that the total amount is not more than 99.9wt%. In one embodiment the total amount of Compound A, Compound B and calcium phosphate is at least 90wt%, or at least 95wt%, or at least 99wt%, or at least 99.5wt%.

### Applications

The scope of this invention is defined by the claims. Any references in the description to methods of treatment refer to the compositions of the present invention for use in a method for treatment of the human (or animal) body.

The present invention may be used as a primer for example when treating hard tissue such as bone or tooth, or metal or synthetic materials. The primer may be used in combination with a stabilizing device such as patch or with a filler. The patch then comprises a layer of the primer or the compound according to the present invention and on said layer is a layer of a composition comprising the reaction product of at least one component A and at least one component B. Component A comprises a compound comprising at least two thiol groups or a disulfide derivative of a compound comprising at least two thiol groups. Component B comprises vinyl reactive groups chosen from vinyl, acrylates, methacrylates, allyl, unsaturated cyclic vinyls, and alkynes. Preferably the Component B comprises vinyl reactive groups chosen from vinyl, allyl and unsaturated cyclic vinyls, and alkynes. This composition is further described in WO2011048077 which is hereby incorporated by reference. The patch may further comprise fibres such as glass fibres, carbon fibres, polyethylene fibres, cellulose fibres, collagen fibres or polypropylene fibres. In one embodiment, the adhesive composite, alternatively with organic fillers, according to the present invention is applied on top of the primer composition prior to applying the patch, supporting material or filler. The adhesive composite may be applied almost directly after application of the primer composition but in order for the primer to adhere to the tissue and to at least partly cure, the adhesive composite may be applied after 10-60 seconds. The primer can be cured via light irradiation before or after the adhesive is applied, in the latter case the adhesive and primer is cured together. Further layers of the adhesive, alternatively with ceramic fillers can be applied together or without fibers and then cured via light irradiation.

Compound A may be selected from the group consisting of pentaerythritol tetrakis(3-mercaptopropionate), trimethylolpropane tris(3-mercaptopropionate), , tris 2-(3-mercaptopropionyloxy)ethyl isocyanurate (TEMPIC), 1,3,5-tris(3-mercaptopropyl)-1,3,5-triazinane-2,4,6-trione, mercaptopropyl methylsiloxane-dimethylsiloxane copolymer,poly (mercaptopropyl) methylsiloxane, 2,2' (ethylenedioxy)diethanethiol, ditiotreitol, Tetraethyleneglycol-bis(3-mercaptopropionate), ethyleneglycol-bis(3-mercaptopropionate), trimethylolpropane diallylether, dipentaerytritolhexakis(3-merkaptopropionate), tetradecane-1,14-dithiol, ( +/-)-trans-1,2-bis (2-mercaptoacetamido) cyclohexane, (E)-S, S '-bis (lomercaptodecyl)-4,4'-(diazene-1,2-diyl) bis (4-cyanopentanethioate), bis(2-mercaptoethyl)sulfone, 2,5-dimercaptomethyl-1,4-dithiane, 1,4-butanediol-bis(3-mercaptopropionate), 1,16-hexadecanedithiol, undecane-1,11-dithiol, heptane-1,7-dithiol, 1,12-dimercaptododecane, octadecane-1,18-dithiol, (5-mercaptomethyl-2,4-dimethyl-phenyl)-methanethiol, (3-mercaptomethyl-5-methyl-phenyl)-methanetho, 1,2-benzenedimethanethiol, (4R, 5R)-4,5-bis (mercaptomethyl)-2,2-dimethyl-1,3-dioxolane, 3-bis (2-mercaptoethylthio)propane, ethanethiol, aceticacidmercapto-1,2,6-hexanetriyl ester, L-1,4-dithiothretol, glycerylthioglycolate, 3,6-dioxa-1,8-octanedithiol,trimethylolpropane-tris(mercaptoacetate) 2,3-butanediol-1,4-dimercapto-pentaerythritol-tetrakis(3-mercaptopropionate), ethanethiol-2,2',2"-nitrilotris, 2,2'-thiodiethanethiol, 1,9-nonanedithiol, 2,2'-oxydiethanethiol, and 10-decanedithiol.

Compound B may be selected from the group consisting of trimethylolpropane diallyl ether, 1,3,5-triallyl-1,3,5-triazine-2,4,6 (1H,3H,5H) -trione (TATATO), 1,3,5-tri(prop-2-yn-1-yl)-1,3,5-triazinane-2,4,6-trione (TPYTATO), b) 1,3,5-tri(hex-5-yn-1-yl)-1,3,5-triazinane-2,4,6-trione (THYTATO), trimethylolpropane diallyl ether, poly(ethylene glycol)diacrylate, poly(ethylene glycol) dimethacrylate andpoly(ethylene glycol) dimaleinimide.

The patch may further comprise ceramic particles or functionalized ceramic particles. In one embodiment the amount of calcium phosphate particles in the patch is 1-50wt%.

In one embodiment the patch applied on top of the primer comprises a fiber mesh and TATATO, TEMPIC and a photo initiator (eg. irgacure 819), or TATATO, TEMPIC, photo initiator (eg. irgacure 819) and hydroxyapatite (HA), or TATATO, TEMPIC, photo initiator (eg. irgacure 819) and functionalized HA, or TATATO, TEMPIC and a photo initiator (eg. Camphorquinone (CQ)).

A filler comprising the present composition or compound may also contain particles of a ceramic such as hydroxyapatite or glass. The filler may comprise between 1 to 99wt% of calcium phosphate particles such as 10wt% or more, or 30wt% or more, or 80wt% or less or 60wt% or less.

The present invention further relates to calcium phosphate material, surface or particles for example, coated with the composition or the compound according to the present invention.

Curing of the composition may be done using any suitable radiation or light source in order to initiate the reaction. This can be but is not limited to a light emitting diode (LED) polymerization lamp (e.g. Bluephase^{®} 20i) emitting light with wavelengths of 385-515 nm with a dominant wavelength of 470 nm and maximum intensity of 2000-2200 mW/cm². If more than one layer of the composition or the compound is used each layer may be cured using different irradiation time and intensity.

The present invention presents an improved method of treating bone or tooth fractures or damages. By applying the primer to the hard tissue better mechanical properties are obtained. No other pre-treatments are necessary such as etching of the tissue surface prior to the application of the patch or coating.

### Kit

The present invention further relates to a kit in order to provide and apply the primer composition and optionally the adhesive composite. The kit contains at least two suitable containers where one of the containers comprises water; and where one container comprises the first compound having at least two thiol groups, the second compound 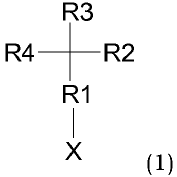
wherein R1 is an alkyl, a dialkyl ether, dialkyl ester, dialkyl urethane or a dialkyl amide group, R2 is an at least one allyl containing group or an at least one alkyne containing group, R3 is hydrogen, an alkyl group or an at least one allyl containing group or an at least one alkyne containing group and R4 is hydrogen, an alkyl group or an at least one allyl containing group or an at least one alkyne containing group;
wherein the first compound or the second compound comprises at least one phosphonic acid containing group X with proviso that when the second compound comprises the at least one phosphonic acid containing group said group is bound to the R1 group.

One of the containers may comprise a third compound or additional compounds comprising at least one allyl containing group or at least one thiol group or at least one alkyne containing group.

The kit further comprises a photo initiator. However in order to avoid reactions in the containers the thiol containing compounds should not be present in the same containers as the allyl containing compounds. The molar ratio between the phosphonic acid groups and the allyl groups in the kit is 0.01 to 0.5.

The kit may further comprise at least two additional containers comprising the components of the adhesive composite i.e. a photoinitiator, a Component A comprising at least two thiol groups, a Component B comprising vinyl reactive groups chosen from vinyl, acrylate, methacrylate, allyl, unsaturated cyclic vinyls, and alkynes and ceramic filler, such as calcium phosphate particles or functionalized calcium phosphate particles. The Component A and Component B may however not be in the same containers in order to avoid reactions in the container.

In one embodiment the containers of the kit are configured to minimize or stop radiation to come in contact with the components or reactants of the containers.

The kit may be in form of one or more at least two compartment syringe. The syringe may comprise a mixing tip. In one embodiment the primer components are arranged in a first at least two compartment syringe and the adhesive composite compounds are arranged in a second at least two compartment syringe.

### EXAMPLES

### Example 1

### Synthesis of Adhesion-Enhancing Components

All reagents and solvents were purchased from commercial sources and used as received unless otherwise stated. 3-(allyloxy)-2-((allyloxy)methyl)-2-methylpropanoic acid (BAPA) was synthesized according to a previously published procedure (P. Antoni, M. J. Robb, L. Campos, M. Montañez, A. Hult, E. Malmström, M. Malkoch, C. J. Hawker, Macromolecules 2010, 43, 6625-6631).

The synthesis of adhesion-enhancing molecules was performed through simplified protocols. The details for some example synthetic approaches are described in the Method of preparing section, in general, the dendritic compounds were prepared by reactions assisted by 1,1'-Carbonyldiimidazole (CDI), resulting in short reaction times, easy purifications and high yields. In brief, BAPA or the BAPA-functionalized dendron was activated with CDI in stoichiometric ratio and subsequently reacted *in-situ* with the corresponding amino-functional reagent. A simple washing affords the BAPA-derivatives in high yields. Some other reactions were required to obtain the functional materials, including Kabachnik-Fields reaction and phosphonate ester cleavage using Trimethylbromosilane (TMBS).

### Example 1.1 - Phn-G1-(ene)₂ (2a)

In a first step, BAPA (1.5 g, 7.0 mmol) was slowly added over a suspension of CDI (1.2 g, 7.0 mmol) in EtOAc (2 M). The mixture was stirred at 50 °C for 1 h.

Diethyl(aminomethyl)phosphonate (1.0 g, 5.8 mmol) was added to the solution and the reaction proceeded at 50°C for 30 minutes. The activated acid was quenched by high stirring with water for 1 h and the mixture was diluted with EtOAc and washed with NaHCOs 10% and NaHSO₄ 10%. The organic phase was dried over MgSO₄, filtered and evaporated to dryness. Further purification through silica chromatography using EtOAc as elution solvent afforded EtPhn-G1-(ene)₂ [C₁₆H₃₀NO₆P (363,4)] as yellow oil (1.3 g, 62% yield).

In a second step, EtPhn-G1-(ene)₂ (1.0 g, 2.8 mmol) was dissolved in 2 mL CHCl₃ and trimethylbromosilane (910 µL, 6.9 mmol) was slowly added at 0°C. The mixture was stirred at r.t. for 18 h and evaporated to dryness. The residue was redissolved in 2 mL MeOH and stirred at r.t. for 2 h. After solvent evaporation, the residue was redissolved in DCM and evaporated. Phn-G1-(ene)₂ **(2a)** [C₁₂H₂₂NO₆P (307,3)] was isolated as white solid (860 mg, 100% yield).

### Example 1.2 - (Phn)₂-EDA-G1-(ene)₂ (2b)

In a first step, BAPA (10.0 g, 0.047 mol) was slowly added over a suspension of CDI (7.8 g, 0.047 mol) in EtOAc (2 M). The mixture was stirred at 50 °C for 1 h and dropwise added to a solution of ethylenediamine (6.2 mL, 0.093 mol) in EtOAc (25 mL) while stirring at 50°C. After 2 h, the mixture was washed with NaHCOs 10% (3x). The organic phase was dried over MgSO₄, filtered and evaporated to dryness. Further purification through silica chromatography using DCM:NEts (99:1) as eluting phase afforded EDA-G1-(ene)₂ [C₁₃H₂₄N₂O₃ (256,3)] as yellow oil (6.0 g, 50% yield).

In a second step, EDA-G1-(ene)₂ (500 mg, 1.95 mmol) and p-formaldehyde (130 mg, 3.90 mmol) were mixed in THF (500 µL). After 1 h at 70°C, dimethylphosphite was added dropwise. The reaction proceeded at 70°C for 18 h. Then, the solvent was removed under vacuum and the mixture was redissolved in CHCl₃ and washed with NaOH 0.1 M (3x). The organic phase was dried over MgSO₄, filtered and evaporated to dryness. (MePhn)₂-EDA-G1-(ene)₂ [C₁₉H₃₈N₂O₉P₂ (500,5)] was isolated as yellow oil (822 mg, 85% yield).

In a third step, (MePhn)₂-EDA-G1-(ene)₂ (500 mg, 1.0 mmol) was dissolved in 1 mL CHCl₃ and trimethylbromosilane (659 µL, 5.0 mmol) was slowly added at 0°C. The mixture was stirred at r.t. for 18 h and evaporated to dryness. The residue was redissolved in 1 mL MeOH and stirred at r.t. for 2 h. After solvent evaporation, the residue was redissolved in DCM and evaporated. (Phn)₂-EDA-G1-(ene)₂ (2b) [C₁₅H₃₀N₂O₉P₂ (444,4)] was isolated as white solid (444 mg, 100% yield).

### Example 2

### Functionalization of hydroxyapatite (HA) particles

(Phn)₂-EDA-G1-(ene)₂ (45 mg, 0.1 mmol) was dissolved in 3 ml MeOH:H₂O (1:4). HA particles (4.5 g) were dispersed in the solution and stirred for 5h. The particles were collected using a filter, rinsed with methanol and evaporated to dryness.

### Example 3

### Example compositions

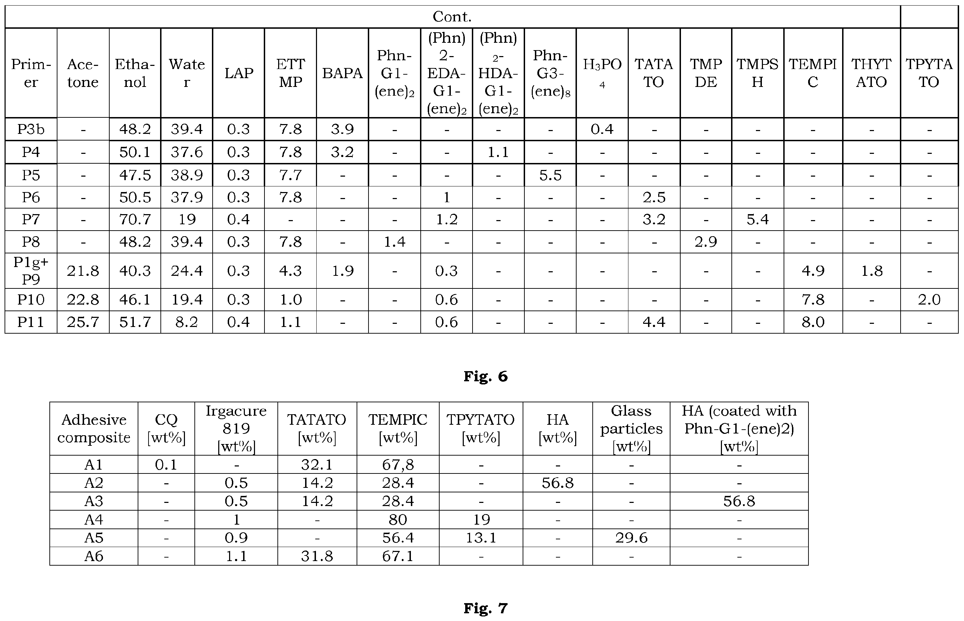

Figure 7 discloses examples primer compositions.

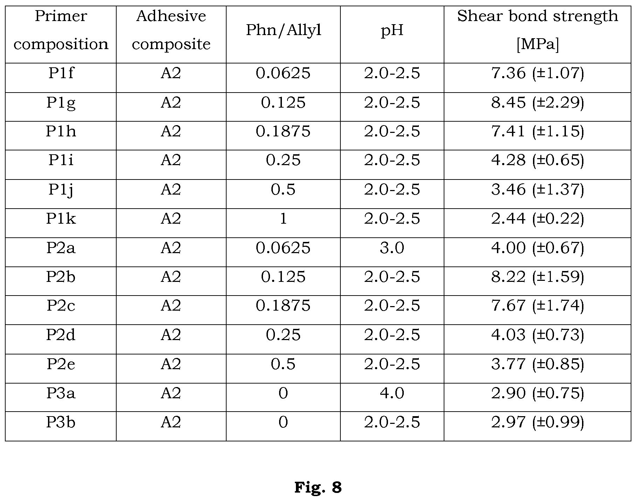

Figure 8, description of example adhesive compositions for FRAP

### Adhesive bond strength

### Method 1. Adhesive shear bond strength: FRAP on bone.

Rectangular bone pieces (4 x 1.5 cm) were polished to obtain even surfaces and finally sanded using P80 sand paper. The pieces were kept wet with m-SBF (modified Simulated Body Fluid). Fiber reinforced adhesive patches were used to glue bone pieces together in pairs by bridging over the gap between the two bone pieces. Two patches per pair was used in a double-lap shear strength mode to form a more even force distribution. The patches were built up by first wiping the wet bone surface with a tissue cloth and then spreading 1 µl of primer solution on the bonding area of 0.5 x 0.5 cm of each bone piece. The primer solution was either let to evaporate until a moist state or dried with gentle air-flow. Six consecutive layers of adhesive matrix surrounding five layers of surgical mesh were built up. Curing was induced using a light emitting diode (LED) polymerization lamp (Bluephase^{®} 20i) intended for light-cured dental materials. Bluephase^{®} 20i emits light with wavelengths of 385-515 nm with a dominant wavelength of 470 nm and maximum intensity of 2000-2200 mW/cm². The first layer was cured with irradiation for 20 s with TURBO mode (2000 mW/cm²), the rest of the layers were irradiated for 10 s with TURBO mode. The patches were submerged in m-SBF directly after final irradiation sequence and stored for 24h at 37°C and then let to cool down to 23°C before testing. Instron 5566 from Instron Korea LLC. with a 10kN load cell and a cross-head speed of 10 mm/min was used in tensile mode. A pre-load of 1 N and a pre-load speed of 2 mm/min were used. The measurements were conducted at 23 °C and a relative humidity of 50 %. The samples were kept wet as long as possible until they were attached to the machine. Data were collected using Bluehill software. Adhesive bond shear strength was calculated from maximum load at break divided by the area of failure. Five specimens of each sample were used.

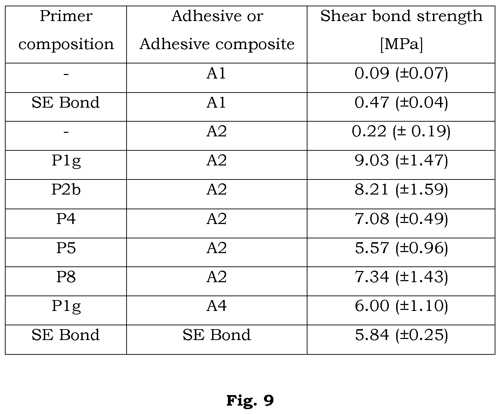

Figure 9. Adhesive shear bond strength with different Phosphonic acid/Allyl ratio and pH: FRAP on bone.

*Key conclusions:* A specific ratio of the adhesion-enhancing group phosphonic acid is needed in a specific ratio range to reach high bond strength. Even though pH of the primer seems to be important it is not enough to just lower the pH with phosphoric acid to reach high bond strength.

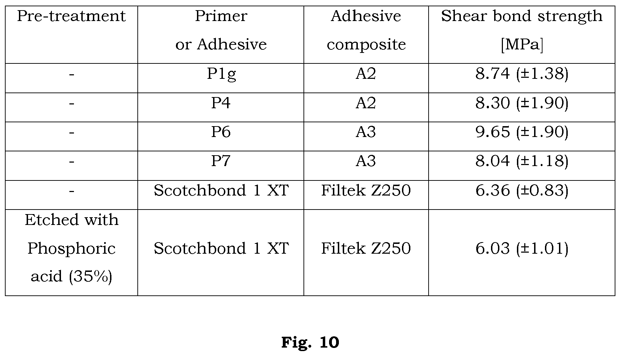

Figure 10. Adhesive shear bond strength with different adhesion enhancing molecules: FRAP on bone.

*Key conclusions:* The new primer compositions increase the bond strength greatly for the adhesive composite. The new primers together with a thiol-ene or thiol-yne adhesive composite display superior bond strength to wet bone compared to commercial dental adhesives.

### Method 2. Adhesive shear bond strength: Adhesive on bone.

Wet rectangular bone pieces (4 x 1.5 cm) were polished to obtain even surfaces and finally sanded using P80 sand paper. The pieces were kept wet with m-SBF. Primer solution was applied over an area of 5x5 mm after wiping away excessive water from the bone surface. The primer was dried with gentle airflow and adhesive composite was applied on the primer treated area. Three layers were applied with 10 s curing with TURBO mode in between each layer to build up a drop on the bone piece. The bone pieces were submerged in m-SBF directly after final irradiation sequence and stored for 24h at 37°C and then let to cool down to 23°C before testing. Instron 5566 from Instron Korea LLC. with a 10kN load cell and a cross-head speed of 5 mm/min was used in compression mode. The bone piece was fixed and the adhesive drop was sheared off by a metal plate. A pre-load of 1 N and a pre-load speed of 2 mm/min were used. The measurements were conducted at 23 °C and a relative humidity of 50 %. The samples were kept wet as long as possible until they were attached to the machine. Data were collected using Bluehill software. Adhesive bond shear strength was calculated from maximum load at break divided by the area of failure. Five specimens of each sample were used.

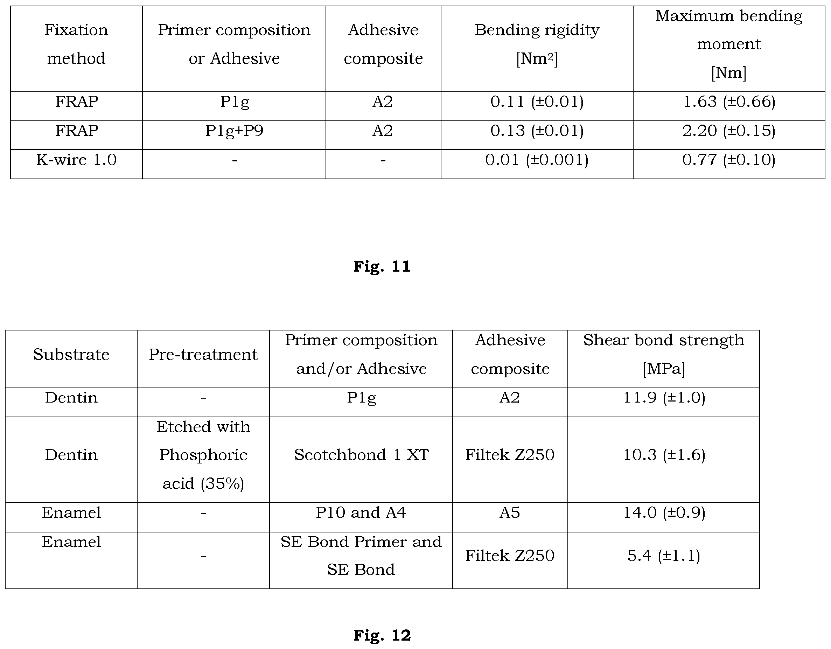

Figure 11. Adhesive shear bond strength: Adhesive on bone.

*Key conclusions:* The new primer compositions together with a thiol-ene adhesive composite display superior bond strength to wet bone compared to a commercial dental restoration system.

### Method 3. Adhesive shear bond strength: Adhesive on porcine bone.

Shear bond strength tests was performed using an Instron 5566 instrument, Instron Korea LLC. A 500 N load cell and a cross-head speed of 5 mm/min in compression mode with a pre-load of 1 N and a pre-load speed of 2 mm/min. The measurements were conducted at 23 °C and a relative humidity of 50 %. Porcine metacarpals where the soft tissue had been removed and the bone cut into two pieces were used as substrates. The bone surface was roughened with an abrasive pin and then wiped with a wet tissue cloth. First a primer was applied in an amount of 4 µl/cm2 and dried with gentle air-flow. Then an adhesive resin was applied as a drop and cured with 20 s of irradiation with Bluephase 20i with TURBO mode (λ = 385-515 nm, 2000 mW/cm2). The bones were kept in wet tissue before and after sample preparation to maintain moist conditions. The dental adhesive reference was applied on both substrates in a similar fashion but adjusted to the intended application procedure from the manufacturers. Five specimens of each sample were tested.

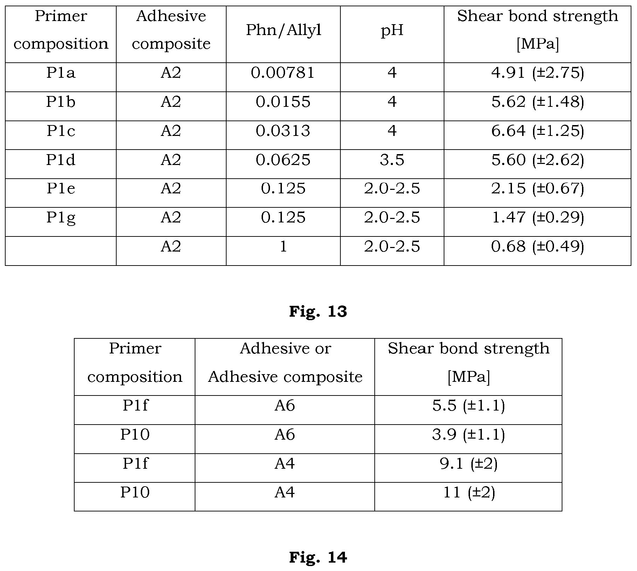

Figure 14. Adhesive shear bond strength: Adhesive on porcine metacarpal.

*Key conclusions:* The thiol-yne adhesive show higher adhesion than the thiol-ene adhesive and with a primer containing a large amount of alkyne monomer and reduced amount of allyl monomer increased the bond strength further for the thiol-yne adhesive

### Method 4. Adhesive shear bond strength: Adhesive on contaminated bone.

Wet rectangular bone pieces (4 x 1.5 cm) were polished to obtain even surfaces and finally sanded using P80 sand paper. The pieces were kept wet with m-SBF. Bovine blood serum was applied onto the bone surfaces and dried with gentle airflow to form a contaminating protein layer. Primer solution was applied over an area of 5x5 mm on the contaminated bone surface and brushed into the surface using a tuft brush. The adhesive composite was applied on the primer treated area. Three layers were applied with 10 s curing with TURBO mode in between each layer to build up a drop on the bone piece. The bone pieces were submerged in m-SBF directly after final irradiation sequence and stored for 24h at 37°C and then let to cool down to 23°C before testing. Instron 5566 from Instron Korea LLC. with a 500 N load cell and a cross-head speed of 5 mm/min was used in compression mode. The bone piece was fixed and the adhesive drop was sheared off by a metal plate. A pre-load of 1 N and a pre-load speed of 2 mm/min were used. The measurements were conducted at 23 °C and a relative humidity of 50 %. The samples were kept wet as long as possible until they were attached to the machine. Data were collected using Bluehill software. Adhesive bond shear strength was calculated from maximum load at break divided by the area of failure. Three specimens of each sample were used.

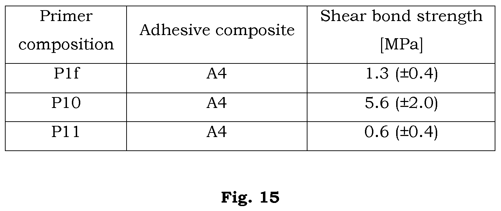

Figure 15. Adhesive shear bond strength: Adhesive on contaminated bone.

*Key conclusions:* The primer composition P10 containing an alkyne component formed a much stronger bond to the contaminated bone surface than primer composition P1f.

### Method 5. FRAP fixation of phalangeal fracture model.

Fracture fixation was tested using the second and the fifth metacarpals of domestic pigs. Surrounding soft tissue and periosteum were removed from the bones. A transverse fracture was created on each bone using a saw. The bones were washed with water and then wrapped in cloth soaked in amoxicillin solution to keep the bones wet and prevent bacterial growth. FRAP technology with primer composition P1b and adhesive composite A2 was used to fixate the fractures. As comparative control fixations with one method with two crossed Kirschner wires 1.0 and one method with Compact hand Locking Strut Plate 1.5 were used. Three-point bending was performed with a distance of 3 mm between the lower contacts in an Instron 5566 (Instron Korea LLC) with a 10 kN load cell and a cross-head speed of 10 mm/min. The measurements were conducted at 23 °C and a relative humidity of 50 %. The samples were kept wet as long as possible until they were attached on the bending apparatus. Data were collected using Bluehill software. Five specimens of each fixation method were tested.

Figure 12. Bending rigidity and maximum bending moment of a FRAP containing primer P1b compared to conventional finger fixations of K-wire 1.0 and Compact hand 1.5.

*Key conclusions:* The new primer systems provides enough bond strength to enable that FRAP fixation gives superior support to K-wires and can even compete with the screw fixated metal plate in the load range of interest.

### Method 6. Adhesive shear bond strength: Adhesive on dentin.

In the first test set-up, roe dear teeth were polished to expose the dentin and obtain even surfaces and finally sanded using P80 sand paper. The teeth were kept wet with m-SBF. Primer solution was applied over an area of 5x5 mm after wiping away excessive water from the tooth surface. The primer was dried with gentle airflow and adhesive composite was applied on the primer treated area. Three layers were applied with 10 s curing with TURBO mode in between each layer to build up a drop on the tooth. The teeth were submerged in m-SBF directly after final irradiation sequence and stored for 24h at 37°C and then let to cool down to 23°C before testing. Instron 5566 from Instron Korea LLC. with a 10kN load cell and a cross-head speed of 5 mm/min was used in compression mode. The tooth was fixed and the adhesive drop was sheared off by a metal plate. A pre-load of 1 N and a pre-load speed of 2 mm/min were used. The measurements were conducted at 23 °C and a relative humidity of 50 %. The samples were kept wet as long as possible until they were attached to the machine. Data were collected using Bluehill software. Adhesive bond shear strength was calculated from maximum load at break divided by the area of failure. Five specimens of each sample were used. The second test substrate was human dental enamel where the surface had been smoothened with a rotary cutter and then wiped with a wet tissue cloth. First a primer was applied in an amount of 4 µl/cm2 and dried with gentle air-flow. Then an adhesive resin was applied as a thin layer and cured with 20 s of irradiation with Bluephase 20i. Then a composite was applied as a drop and cured with 20 s of irradiation with Bluephase 20i. The teeth were kept in water before and after sample preparation to maintain wet conditions. The dental adhesive reference was applied on both substrates in a similar fashion but adjusted to the intended application procedure from the manufacturers. Five specimens of each sample were tested.

Figure 13. Adhesive shear bond strength: Adhesive on dentin and enamel.

*Key conclusions:* The new primer together with a thiol-ene adhesive composite display at least as high bond strength to wet dentine compared to a commercial dental restoration system. The primer composition constituting of alkyne to a major part together with an thiol-yne adhesive as second layer and an thiol-yne adhesive composite as a third layer displayed extremely good adhesion to human enamel compared to a commercial dental restoration system.

### Method 7. Adhesive shear bond strength: Adhesive on steel.

Primer solution was applied on steel over an area of 5x5 mm. The primer was dried with gentle airflow and adhesive composite was applied on the primer treated area. Three layers were applied with 10 s curing with TURBO mode in between each layer to build up a drop on the steel plate. The steel plate was submerged in m-SBF directly after final irradiation sequence and stored for 24h at 37°C and then let to cool down to 23°C before testing. Instron 5566 from Instron Korea LLC. with a 10kN load cell and a cross-head speed of 5 mm/min was used in compression mode. The metal plate was fixed and the adhesive drop was sheared off by a second metal plate. A pre-load of 1 N and a pre-load speed of 2 mm/min were used. The measurements were conducted at 23°C and a relative humidity of 50%. The samples were kept wet as long as possible until they were attached to the machine. Data were collected using Bluehill software. Adhesive bond shear strength was calculated from maximum load at break divided by the area of failure. Five specimens of each sample were used.

Figure 14. Adhesive shear bond strength with different Phosphonic acid/Allyl ratio and pH: Adhesive on steel.

*Key conclusions:* A specific ratio of phosphonic acid and allyl is needed to reach high bond strength. Less phosphonic acid is needed on steel compared to bone to reach high bond strength.

## Claims

1. An aqueous composition comprising
a first compound having at least two thiol groups;
a second compound having the general structure of 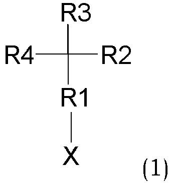
wherein R1 is an alkyl, a dialkyl ether, dialkyl ester, dialkyl urethane or a dialkyl amide group, R2 is an at least one allyl or alkyne containing group, R3 is hydrogen, an alkyl group or an at least one allyl containing group, or an alkyne group or an at least one alkyne containing group, and R4 is hydrogen, an alkyl group or an at least one allyl containing group, or an alkyne group or an at least one alkyne containing group;
wherein the first compound or the second compound comprises at least one phosphonic acid containing group X with the proviso that when the second compound comprises the at least one phosphonic acid containing group said group is bound to the R1 group; and
a third compound or additional compounds comprising at least one alkyne containing group;
preferably also a fourth compound or additional compounds comprising at least one allyl containing group or at least one thiol containing group or at least one alkyne; and
a photo initiator.

2. The aqueous composition according to claim 1 wherein the pH of the composition is in the range of 1.5 to 4.

3. The aqueous composition according to claim 1 or 2, wherein the molar ratio between the phosphonic acid groups and the allyles groups in the composition is 0.01 to 4 with alkynes as the third compound.

4. The aqueous composition according to any of claims 1 or 2 wherein the molar ratio between the phosphonic acid groups and the thiols groups is 0.01 to 0.5 with alkynes as the third compound and wherein the molar ratio between the phosphonic acid groups and the alkyne groups is 0.02 to 1 with alkynes as third compound or as second and third compounds.

5. The aqueous composition according to any of claims 1 or 2 wherein the molar ratio between the phosphonic acid groups and the alkyne groups is 0.02 to 1 with alkynes as third compound or as second and third compounds.

6. The aqueous composition according to any one of the preceding claims
wherein the second compound has the general structure (1) and wherein X is a phosphonic acid group, or wherein the second compound preferably is a dendrimer having four or more allyl groups or two or more alkyne groups, wherein the second compound preferably has the general structure of 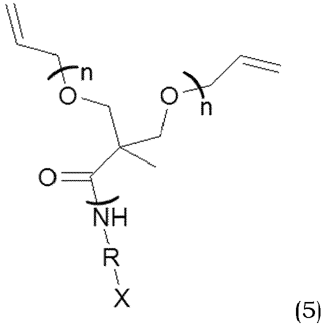
wherein n is 1 or higher, R is an alkyl group and X is a phosphonic acid containing group.

7. The aqueous composition according to any one of the preceding claims wherein each of the at least one allyl containing group is an alkyl allyl group containing two or more allyl groups, or each of the at least one alkyne containing group is an alkyl alkyne group containing one or more alkyne groups, or each of the at least one allyl containing group is a dialkyl ether allyl group containing two or more allyl groups or each of the at least one alkyne containing group is a dialkyl ether alkyne group containing one or more alkyne groups.

8. The aqueous composition according to any of the preceding claims wherein the third compound or additional compounds is a dendrimer having two or more alkyne groups.

9. The aqueous composition according to any of the preceding claims wherein the phosphonic acid containing group of the second compound has the structure of: 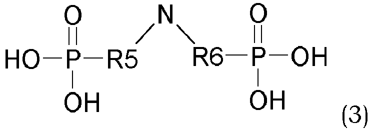 wherein R5 and R6 are individually an alkyl group such as a C1-C10 alkyl groups such as a methylene or an ethylene group.

10. The aqueous composition according to any of the preceding claims wherein the second compound comprises at least one phosphonic acid containing group and wherein the composition comprises a third compound having the general structure of 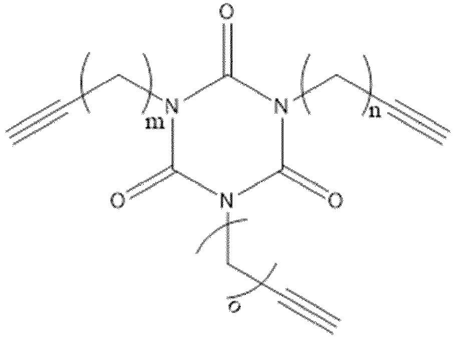 wherein m, n and o is 1 or higher, wherein the total amount of the second compound and third compound preferably is 1-10wt%, such as 2-6wt%, or 4-5wt%.

11. The aqueous composition according to any one of the preceding claims wherein the first compound is selected from tris 2-(3-mercaptopropionyloxy)ethyl isocyanurate (TEMPIC), ethoxylated-trimethylolpropane tri-3-mercaptopropionate (ETTMP), trimethylolpropane tri-3-mercaptopropionate, thiolated polyethylene glycol), 1,3,5-tris(3-mercaptopropyl)-1,3,5-triazinane-2,4,6-trione (TTTSH) and ethoxylated-trimethylolpropane tri-thiol (ETTT) and thiolated multi armed poly(ethylene glycol) and wherein the third compound is preferably selected from 1,3,5-tri(prop-2-yn-1-yl)-1,3,5-triazinane-2,4,6-trione (TPYTATO), 1,3,5-tri(hex-5-yn-1-yl)-1,3,5-triazinane-2,4,6-trione (THYTATO), Bis(propynyloxymethyl)propanoic acid (BPPA).

12. The aqueous composition according to any one of the preceding claims wherein the composition further comprises ethanol or acetone preferably in an amount of 10-90wt% such as 40-60wt% of the total weight of the composition.

13. A patch comprising
a first layer or primer layer comprising the composition according to any one of claims 1 to 12;
a second layer of adhesive comprising photoinitiator, a Component A comprising at least two thiol groups, a Component B comprising vinyl reactive groups chosen from vinyl, acrylate, methacrylate, allyl and unsaturated cyclic vinyls, or alkynes; and
Optionally a third layer comprising Component A comprising at least two thiol groups, a Component B comprising vinyl reactive groups chosen from vinyl, acrylate, methacrylate, allyl and unsaturated cyclic vinyls, or alkynes.

14. A calcium phosphate material having a coating comprising the composition according to any one of claims 1 to 12.

15. A kit comprising at least two containers wherein
any one container in the kit can contain any of
water;
a first compound having at least two thiol groups;
a second compound having the general structure of 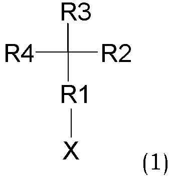
wherein R1 is an alkyl, a dialkyl ether, dialkyl ester, dialkyl urethane or a dialkyl amide group, R2 is an at least one allyl or alkyne containing group, R3 is hydrogen, an alkyl group or an at least one allyl containing group, or an alkyne group or an at least one alkyne containing group, and R4 is hydrogen, an alkyl group or an at least one allyl containing group, or an alkyne group or an at least one alkyne containing group;
wherein the first compound or the second compound comprises at least one phosphonic acid containing group X with proviso that when the second compound comprises the at least one phosphonic acid containing group said group is bound to the R1 group;
a third compound or additional compounds comprising at least one alkyne containing group;
and a photo initiator.

## Patentansprüche

1. Wässrige Zusammensetzung, umfassend
eine erste Verbindung mit wenigstens zwei Thiolgruppen;
eine zweite Verbindung mit der allgemeinen Struktur 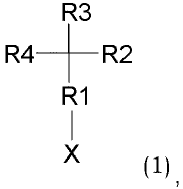
wobei R1 eine Alkyl-, Dialkylether-, Dialkylester-, Dialkylurethan- oder Dialkylamidgruppe ist, R2 eine wenigstens ein Allyl oder Alkin enthaltende Gruppe ist, R3 Wasserstoff, eine Alkylgruppe oder eine wenigstens ein Allyl enthaltende Gruppe oder eine Alkingruppe oder eine wenigstens ein Alkin enthaltende Gruppe ist und R4 Wasserstoff, eine Alkylgruppe oder eine wenigstens ein Allyl enthaltende Gruppe oder eine Alkingruppe oder eine wenigstens ein Alkin enthaltende Gruppe ist;
wobei die erste Verbindung oder die zweite Verbindung wenigstens eine phosphonsäurehaltige Gruppe X umfasst, mit der Maßgabe, dass, wenn die zweite Verbindung die wenigstens eine phosphonsäurehaltige Gruppe umfasst, diese Gruppe an die Gruppe R1 gebunden ist; und
eine dritte Verbindung oder zusätzliche Verbindungen, die wenigstens eine alkinhaltige Gruppe umfassen;
vorzugsweise auch eine vierte Verbindung oder zusätzliche Verbindungen, die wenigstens eine allylhaltige Gruppe oder wenigstens eine thiolhaltige Gruppe oder wenigstens ein Alkin umfassen; und
einen Photoinitiator.

2. Wässrige Zusammensetzung nach Anspruch 1, wobei der pH-Wert der Zusammensetzung im Bereich von 1,5 bis 4 liegt.

3. Wässrige Zusammensetzung nach Anspruch 1 oder 2, wobei das Molverhältnis zwischen den Phosphonsäuregruppen und den Allylgruppen in der Zusammensetzung 0,01 bis 4 mit Alkinen als dritter Verbindung beträgt.

4. Wässrige Zusammensetzung nach einem der Ansprüche 1 oder 2, wobei das Molverhältnis zwischen den Phosphonsäuregruppen und den Thiolgruppen 0,01 bis 0,5 mit Alkinen als dritter Verbindung beträgt und wobei das Molverhältnis zwischen den Phosphonsäuregruppen und den Alkingruppen 0,02 bis 1 mit Alkinen als dritter Verbindung oder als zweiter und dritter Verbindung beträgt.

5. Wässrige Zusammensetzung nach einem der Ansprüche 1 oder 2, wobei das Molverhältnis zwischen den Phosphonsäuregruppen und den Alkingruppen 0,02 bis 1 mit Alkinen als dritter Verbindung oder als zweiter und dritter Verbindung beträgt.

6. Wässrige Zusammensetzung nach einem der vorhergehenden Ansprüche, wobei die zweite Verbindung die allgemeine Struktur (1) aufweist und wobei X eine Phosphonsäuregruppe ist oder wobei die zweite Verbindung vorzugsweise ein Dendrimer mit vier oder mehr Allylgruppen oder zwei oder mehr Alkingruppen ist, wobei die zweite Verbindung vorzugsweise die allgemeine Struktur 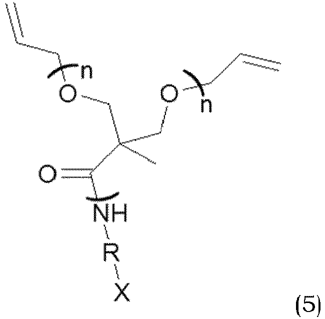 aufweist, wobei n gleich 1 oder höher ist, R eine Alkylgruppe ist und X eine phosphonsäurehaltige Gruppe ist.

7. Wässrige Zusammensetzung nach einem der vorhergehenden Ansprüche, wobei jede der wenigstens einen allylhaltigen Gruppe eine Alkylallylgruppe ist, die zwei oder mehr Allylgruppen enthält, oder jede der wenigstens einen alkinhaltigen Gruppe eine Alkylalkingruppe ist, die eine oder mehr Alkingruppen enthält, oder jede der wenigstens einen allylhaltigen Gruppe eine Dialkyletherallylgruppe ist, die zwei oder mehr Allylgruppen enthält, oder jede der wenigstens einen alkinhaltigen Gruppe eine Dialkyletheralkingruppe ist, die eine oder mehr Alkingruppen enthält.

8. Wässrige Zusammensetzung nach einem der vorhergehenden Ansprüche, wobei die dritte Verbindung oder zusätzliche Verbindungen ein Dendrimer mit zwei oder mehr Alkingruppen sind.

9. Wässrige Zusammensetzung nach einem der vorhergehenden Ansprüche, wobei die phosphonsäurehaltige Gruppe der zweiten Verbindung die Struktur: 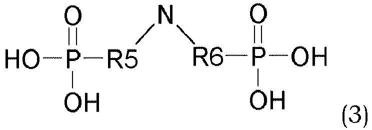 aufweist, wobei R5 und R6 einzeln eine Alkylgruppe wie eine C1-C10-Alkylgruppe wie zum Beispiel eine Methylen- oder eine Ethylengruppe sind.

10. Wässrige Zusammensetzung nach einem der vorhergehenden Ansprüche, wobei die zweite Verbindung wenigstens eine phosphonsäurehaltige Gruppe umfasst und wobei die Zusammensetzung eine dritte Verbindung mit der allgemeinen Struktur 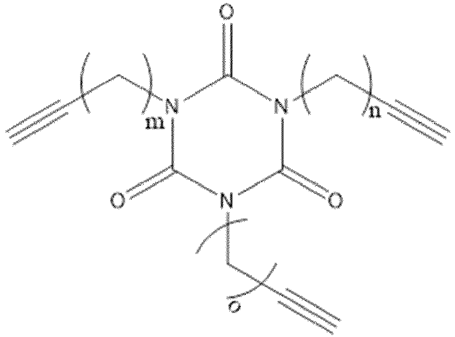 umfasst, wobei m, n und o gleich 1 oder höher sind, wobei die Gesamtmenge der zweiten Verbindung und der dritten Verbindung vorzugsweise 1-10 Gew.-% wie zum Beispiel 2-6 Gew.-% oder 4-5 Gew.-% beträgt.

11. Wässrige Zusammensetzung nach einem der vorhergehenden Ansprüche, wobei die erste Verbindung ausgewählt ist aus Tris-2-(3-mercaptopropionyloxy)ethyl-isocyanurat (TEMPIC), ethoxyliertem Trimethylolpropan-tri-3-mercaptopropionat (ETTMP), Trimethylolpropan-tri-3-mercaptopropionat, thioliertem Poly(ethylenglykol), 1,3,5-Tris(3-mercaptopropyl)-1,3,5-triazinan-2,4,6-trion (TTTSH) und ethoxyliertem Trimethylolpropantrithiol (ETTT) und thioliertem mehrarmigen Poly(ethylenglykol) und wobei die dritte Verbindung vorzugsweise ausgewählt ist aus 1,3,5-Tri(prop-2-in-1-yl)-1,3,5-triazinan-2,4,6-trion (TPYTATO), 1,3,5-Tri(hex-5-in-1-yl)-1,3,5-triazinan-2,4,6-trion (THYTATO), Bis(propinyloxymethyl)propansäure (BPPA).

12. Wässrige Zusammensetzung nach einem der vorhergehenden Ansprüche, wobei die Zusammensetzung ferner Ethanol oder Aceton vorzugsweise in einer Menge von 10-90 Gew.-% wie zum Beispiel 40-60 Gew.-% des Gesamtgewichts der Zusammensetzung enthält.

13. Pflaster, umfassend
eine erste Schicht oder Primerschicht, umfassend die Zusammensetzung nach einem der Ansprüche 1 bis 12;
eine zweite Schicht aus Kleber, umfassend Photoinitiator, eine Komponente A, die wenigstens zwei Thiolgruppen umfasst, eine Komponente B, die vinylreaktive Gruppen, ausgewählt aus Vinyl, Acrylat, Methacrylat, Allyl und ungesättigten cyclischen Vinylen, oder Alkine umfasst, und
optional eine dritte Schicht, umfassend eine Komponente A, die wenigstens zwei Thiolgruppen umfasst, eine Komponente B, die vinylreaktive Gruppen, ausgewählt aus Vinyl, Acrylat, Methacrylat, Allyl und ungesättigten cyclischen Vinylen, oder Alkine umfasst.

14. Calciumphosphatmaterial, das eine Beschichtung aufweist, die die Zusammensetzung nach einem der Ansprüche 1 bis 12 umfasst.

15. Kit, das wenigstens zwei Behälter umfasst, wobei
ein beliebiger Behälter in dem Kit Beliebiges enthalten kann von:
Wasser;
einer ersten Verbindung mit wenigstens zwei Thiolgruppen;
einer zweiten Verbindung mit der allgemeinen Struktur 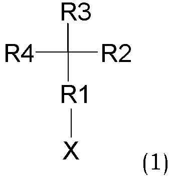 ,
wobei R1 eine Alkyl-, Dialkylether-, Dialkylester-, Dialkylurethan- oder Dialkylamidgruppe ist, R2 eine wenigstens ein Allyl oder Alkin enthaltende Gruppe ist, R3 Wasserstoff, eine Alkylgruppe oder eine wenigstens ein Allyl enthaltende Gruppe oder eine Alkingruppe oder eine wenigstens ein Alkin enthaltende Gruppe ist und R4 Wasserstoff, eine Alkylgruppe oder eine wenigstens ein Allyl enthaltende Gruppe oder eine Alkingruppe oder eine wenigstens ein Alkin enthaltende Gruppe ist;
wobei die erste Verbindung oder die zweite Verbindung wenigstens eine phosphonsäurehaltige Gruppe X umfasst, mit der Maßgabe, dass, wenn die zweite Verbindung die wenigstens eine phosphonsäurehaltige Gruppe umfasst, diese Gruppe an die Gruppe R1 gebunden ist;
einer dritten Verbindung oder zusätzlichen Verbindungen, die wenigstens eine alkinhaltige Gruppe umfassen;
und einem Photoinitiator.

## Revendications

1. Composition aqueuse comprenant
un premier composé ayant au moins deux groupes thiol ;
un deuxième composé ayant la structure générale de 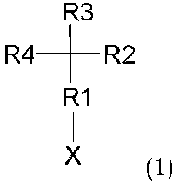
dans laquelle R1 est un alkyle, un dialkyl éther, un dialkyl ester, un dialkyl uréthane ou un groupe dialkyl amide, R2 est un groupe contenant au moins un allyle ou un alcyne, R3 est un hydrogène, un groupe alkyle ou un groupe contenant au moins un allyle, ou un groupe alcyne ou un groupe contenant au moins un groupe alcyne, et R4 est un hydrogène, un groupe alkyle ou un groupe contenant au moins un allyle, ou un groupe alcyne ou un groupe contenant au moins un groupe alcyne ;
dans laquelle le premier composé ou le deuxième composé comprend au moins un groupe X contenant un acide phosphonique à condition que, lorsque le deuxième composé comprend l'au moins un groupe contenant un acide phosphonique, ledit groupe soit lié au groupe R1 ; et
un troisième composé ou des composés supplémentaires comprenant au moins un groupe contenant un alcyne ;
de préférence également un quatrième composé ou des composés supplémentaires comprenant au moins un groupe contenant un allyle ou au moins un groupe contenant un thiol ou au moins un alcyne ; et
un photoinitiateur.

2. Composition aqueuse selon la revendication 1 dans laquelle le pH de la composition est dans la plage de 1,5 à 4.

3. Composition aqueuse selon la revendication 1 ou 2, dans laquelle le rapport molaire entre les groupes acide phosphonique et les groupes allyle dans la composition est de 0,01 à 4 avec les alcynes en tant que troisième composé.

4. Composition aqueuse selon l'une quelconque des revendications 1 ou 2 dans laquelle le rapport molaire entre les groupes acide phosphonique et les groupes thiol est de 0,01 à 0,5 avec les alcynes en tant que troisième composé et dans laquelle le rapport molaire entre les groupes acide phosphonique et les groupes alcyne est de 0,02 à 1 avec les alcynes en tant que troisième composé ou en tant que deuxième et troisième composés.

5. Composition aqueuse selon l'une quelconque des revendications 1 ou 2 dans laquelle le rapport molaire entre les groupes acide phosphonique et les groupes alcyne est de 0,02 à 1 avec les alcynes en tant que troisième composé ou en tant que deuxième et troisième composés.

6. Composition aqueuse selon l'une quelconque des revendications précédentes dans laquelle le deuxième composé a la structure générale (1) et dans laquelle X est un groupe acide phosphonique, ou dans laquelle le deuxième composé est de préférence un dendrimère ayant quatre groupes allyle ou plus ou deux groupes alcyne ou plus, dans laquelle le deuxième composé a de préférence la formule générale de 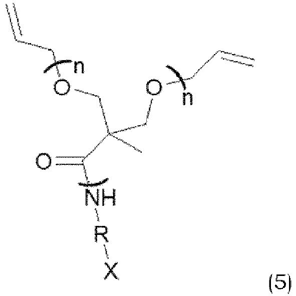 dans laquelle n est 1 ou plus, R et un groupe alkyle et X est un groupe contenant un acide phosphonique.

7. Composition aqueuse selon l'une quelconque des revendications précédentes dans laquelle chacun de l'au moins un groupe contenant un allyle est un groupe alkyl allyle contenant deux groupes allyle ou plus, ou dans laquelle chacun de l'au moins un groupe contenant un alcyne est un groupe alkyl alcyne contenant un ou plusieurs groupes alcyne, ou chacun de l'au moins un groupe contenant un groupe allyle est un groupe dialkyl éther allyle contenant deux groupes allyle ou plus ou chacun de l'au moins un groupe contenant un alcyne est un groupe dialkyl éther alcyne contenant un ou plusieurs groupes alcyne.

8. Composition aqueuse selon l'une quelconque des revendications précédentes dans laquelle le troisième composé ou les composés supplémentaires sont un dendrimère ayant deux groupes alcyne ou plus.

9. Composition aqueuse selon l'une quelconque des revendications précédentes dans laquelle le groupe contenant un acide phosphonique du deuxième composé a la structure de : 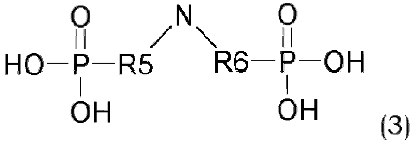 dans laquelle R5 et R6 sont individuellement un groupe alkyle tel qu'un groupe alkyle en C1-C10 tel qu'un groupe méthylène ou éthylène.

10. Composition aqueuse selon l'une quelconque des revendications précédentes dans laquelle le deuxième composé comprend au moins un groupe contenant un acide phosphonique et dans laquelle la composition comprend un troisième groupe ayant la structure générale de 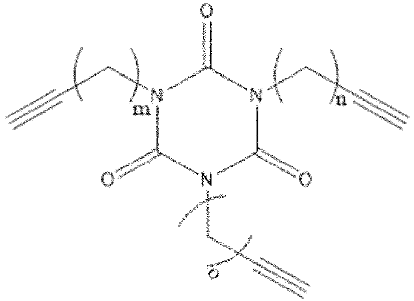 dans laquelle m, n et o sont 1 ou plus, dans laquelle la quantité totale du deuxième et du troisième composé est de préférence de 1 à 10 % en poids, telle que 2 à 6 % en poids, ou de 4 à 5 % en poids.

11. Composition aqueuse selon l'une quelconque des revendications précédentes dans laquelle le premier composé est sélectionné parmi l'isocyanurate de tris 2-(3-mercaptopropionyloxy)éthyle (TEMPIC), le tri-3-mercaptopropionate de triméthylolpropane éthoxylé (ETTMP), le tri-3-mercaptopropionate de triméthylolpropane, le poly(éthylène glycol) thiolé, la 1,3,5-tris(3-mercaptopropyl)-1,3,5-triazine-2,4,6-trione (TTTSH) et le tri-thiol de triméthylolpropane éthoxylé (ETTT) et le poly(éthylène glycol) thiolé à ramifications multiples et dans laquelle le troisième composé est de préférence sélectionné parmi la 1,3,5-tri(prop-2-yn-1-yl)-1,3,5-triazinane-2,4,6-trione (TPYTATO), la 1,3,5-tri(hex-5-yn-1-yl)-1,3,5-triazinane-2,4,6-trione (THYTATO), l'acide bis(propynyloxyméthyl)propanoïque (BPPA).

12. Composition aqueuse selon l'une quelconque des revendications précédentes dans laquelle la composition comprend en outre de l'éthanol ou de l'acétone de préférence en une quantité de 10 à 90 % en poids telle que 40 à 60 % du poids du poids total de la composition.

13. Patch comprenant
une première couche ou couche d'apprêt comprenant la composition selon l'une quelconque des revendications 1 à 12 ;
une deuxième couche d'adhésif comprenant un photoinitiateur, un composant A comprenant au moins deux groupes thiol, un composant B comprenant des groupes réactifs vinyle sélectionnés parmi un vinyle, un acrylate, un méthacrylate, un allyle et des vinyles cycliques insaturés, ou des alcynes ; et
facultativement une troisième couche comprenant le composant A comprenant au moins deux groupes thiol, un composant B comprenant des groupes réactifs vinyle sélectionnés parmi un vinyle, un acrylate, un méthacrylate, un allyle et des vinyles cycliques insaturés, ou des alcynes.

14. Matériau de phosphate de calcium ayant un revêtement comprenant la composition selon l'une quelconque des revendications 1 à 12.

15. Kit comprenant au moins deux contenants dans lequel un contenant quelconque du kit peut contenir l'un quelconque parmi
de l'eau ;
un premier composé ayant au moins deux groupes thiol ;
un deuxième composé ayant la structure générale de 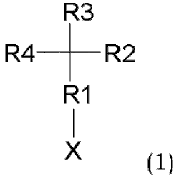
dans lequel R1 est un alkyle, un dialkyl éther, un dialkyl ester, un dialkyl uréthane ou un groupe dialkyl amide, R2 est un groupe contenant au moins un allyle ou un alcyne, R3 est un hydrogène, un groupe alkyle ou un groupe contenant au moins un allyle, ou un groupe alcyne ou un groupe contenant au moins un groupe alcyne, et R4 est un hydrogène, un groupe alkyle ou un groupe contenant au moins un allyle, ou un groupe alcyne ou un groupe contenant au moins un groupe alcyne ;
dans lequel le premier composé ou le deuxième composé comprend au moins un groupe X contenant un acide phosphonique à condition que, lorsque le deuxième composé comprend l'au moins un groupe contenant un acide phosphonique, ledit groupe soit lié au groupe R1 ;
un troisième composé ou des composés supplémentaires comprenant au moins un groupe contenant un alcyne ;
et un photoinitiateur.
